(19) Europäisches Patentamt
European Patent Office
Office européen des brevets

(11) Publication number: **0 291 916 B1**

(12) **EUROPEAN PATENT SPECIFICATION**

(45) Date of publication of patent specification: **12.08.92**

(51) Int. Cl.[5]: **C07D 277/24**, C07D 277/64, C07D 339/00, C07D 263/58, C07D 231/54, C07D 471/04, C07D 239/72, C07D 215/14, C07D 213/30, A61K 31/425

(21) Application number: **88107851.3**

(22) Date of filing: **17.05.88**

The file contains technical information submitted after the application was filed and not included in this specification

(54) **Dithioacetal compounds, processes for preparation thereof and pharmaceutical composition comprising the same.**

(30) Priority: **19.05.87 GB 8711802**

(43) Date of publication of application: **23.11.88 Bulletin 88/47**

(45) Publication of the grant of the patent: **12.08.92 Bulletin 92/33**

(84) Designated Contracting States: **AT BE CH DE ES FR GB GR IT LI LU NL SE**

(56) References cited:
**EP-A- 0 110 405** **EP-A- 0 113 587**
**EP-A- 0 190 722** **DE-A- 2 928 037**
**US-A- 4 102 858** **US-A- 4 535 164**

**CHEMICAL ABSTRACTS, vol. 104, no. 5, February 3, 1986, Columbus, Ohio, USA; SMITH KLINE BECKMAN CORP.: "Lenkotriene antagonists", page 528, column 1, abstract-no. 33 866f**

(73) Proprietor: **FUJISAWA PHARMACEUTICAL CO., LTD.**
**3, Doshomachi 4-chome Higashi-ku**
**Osaka-shi Osaka 541(JP)**

(72) Inventor: **Oku, Teruo**
**17-1, Umezono 2-chome**
**Tsukuba-shi(JP)**
Inventor: **Kawai, Yoshio**
**15-2, Umezono 2-chome**
**Tsukuba-shi(JP)**
Inventor: **Kayakiri, Hiroshi**
**5-4, Umezono 2-chome**
**Tsukuba-shi(JP)**
Inventor: **Kuratani, Kazuyoshi**
**2-25-10, Matsushiro**
**Tsukuba-shi(JP)**
Inventor: **Hashimoto, Masashi**
**11-6, Takezono 2-chome**
**Tsukuba-shi(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

CHEMICAL ABSTRACTS, vol. 85, no. 1, July 5, 1976, Columbus, Ohio, USA; NAGAKURA I. et al.: "2-(4-chlorophenyl)-3-substituted tetrahydro-1,3-thiazin-4-one 1,1 dioxides", page 457, column 1, abstract-no. 5 655g

(74) Representative: Türk, Gille, Hrabal
Brucknerstrasse 20
W-4000 Düsseldorf 13(DE)

## Description

This invention relates to new dithioacetal compounds of formula (I) following below and pharmaceutically acceptable salts thereof, to their preparation and use. More particularly, it relates to new dithioacetal compounds of formula (I) and pharmaceutically acceptable salts thereof which have 5-lipoxygenase-inhibiting activity, to processes for the preparation thereof, to their use in or for preparing medicaments and pharmaceutical compositions comprising the same.

From EP-A-0 190 722 and EP-A-0 110 405 dihydro- and tetrahydroquinoline and -naphthalene derivatives of a similar basic structure are already known which are lipoxygenase inhibitors possessing antiinflammatory and anti-allergic activities. However, their lipoxygenase-inhibiting activities do not meet modern requirements.

Therefore, the object of the present invention is to find new compounds having an even higher lipoxygenase-inhibiting activity.

Surprisingly, it has been found that this object of the present invention can be achieved by novel dithioacetal compounds of the following general formula (I).

According to a first aspect the present invention relates to new dithioacetal compounds having the general formula:

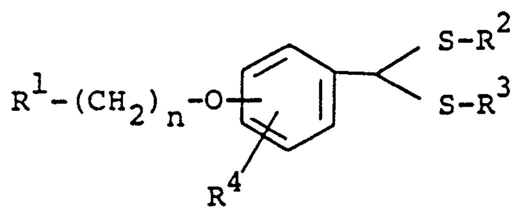

(I)

wherein

$R^1$ is $(C_1\text{-}C_6)$alkyl, di-$(C_1\text{-}C_6)$alkylamino, aryl selected from the group consisting of phenyl, tolyl, xylyl or naphthyl, or heterocyclic group which may have one or more substituents selected from halogen, $(C_1\text{-}C_6)$-alkyl, $(C_1\text{-}C_6)$alkoxy and phenyl, the heterocyclic group being selected from the group consisting of an unsaturated 3- to 8-membered monocyclic heterocyclic group containing 1 to 4 nitrogen atoms selected from the group consisting of pyrrolyl, pyrrolinyl, imidazolyl, pyrazolyl, pyridyl and its N-oxide, dihydropyridyl, pyrimidyl, pyrazinyl, pyridazinyl, triazolyl, a 3- to 8-membered monocyclic heterocyclic group containing at least one sulfur atom and at least one nitrogen atom selected from the group consisting of thiazolyl, isothiazolyl, thiadiazolyl, a polycyclic heterocyclic group containing at least one nitrogen atom selected from the group consisting of indolyl, isoindolyl, indolizinyl, benzimidazolyl, quinolyl, isoquinolyl, quinazolinyl, imidazo(1,2-a)pyridyl, a polycyclic heterocyclic group containing at least one sulfur atom and at least one nitrogen atom selected from the group consisting of benzothiazolyl, benzothiadiazolyl, a polycyclic heterocyclic group containing at least one oxygen atom selected from the group consisting of benzofuranyl, isobenzofuranyl, a polycyclic heterocyclic group containing at least one oxygen atom and at least one nitrogen atom selected from the group consisting of benzoxazolyl, benzoxadiazolyl,

$R^2$ and $R^3$ are each $(C_1\text{-}C_6)$alkyl, aryl or ar-$(C_1\text{-}C_6)$alkyl whereby aryl and the aryl moiety are selected from the group consisting of phenyl, tolyl, xylyl or naphthyl, or $R^2$ and $R^3$ are together to form 1,3-dithian ring,

$R^4$ is hydrogen or $(C_1\text{-}C_6)$alkoxy,

and

n is 0 or an integer of 1 to 4, and pharmaceutical acceptable salts thereof.

Preferred embodiments of the claimed compounds are:

(a) a compound of formula (I), wherein
$R^1$ is a heterocyclic group as defined above,
n is 1,
$R^2$ and $R^3$ are each $(C_1\text{-}C_6)$alkyl, and
$R^4$ is hydrogen;

(b) a compound of the formula

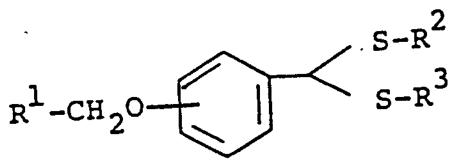

wherein $R^1$ is a heterocyclic group as defined above which may have one or more substituents selected from halogen, $(C_1\text{-}C_6)$alkyl, $(C_1\text{-}C_6)$alkoxy and phenyl, and $R^2$ and $R^3$ are each $(C_1\text{-}C_6)$alkyl;

(c) a compound of the formula:

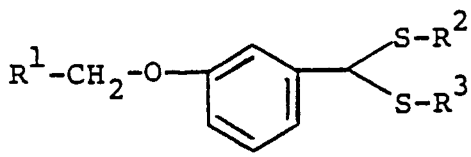

wherein $R^1$ is unsaturated benzene-fused 5- or 6-membered heterocyclic group containing one or two nitrogen atoms as defined above, and $R^2$ and $R^3$ are each $(C_1\text{-}C_6)$alkyl;

(d) a compound of the above formula, wherein $R^1$ is quinolyl and $R^2$ and $R^3$ are each propyl;

(e) a compound of the above formula, wherein $R^1$ is a heterocyclic group of the formula

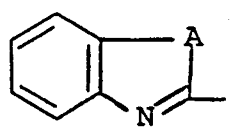

wherein A is

$$-\overset{H}{N}-,$$

-O- or -S- and $R^2$ and $R^3$ are each $(C_1\text{-}C_6)$-alkyl; and

(f) a compound as defined above wherein $R^1$ is benzothiazolyl and $R^2$ and $R^3$ are each propyl.

According to a second aspect the present invention relates to a process for preparation of compounds of general formula (I) as defined above which comprises reacting a compound of the following formula or its salt:

$R^1\text{-}(CH_2)_n\text{-}Y$      (II)

with a compound of the following formula or its salt :

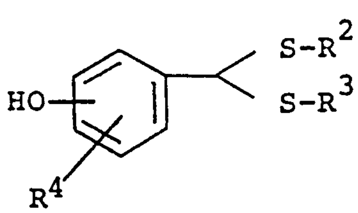

(III)

to give a compound of the following formula or its salt :

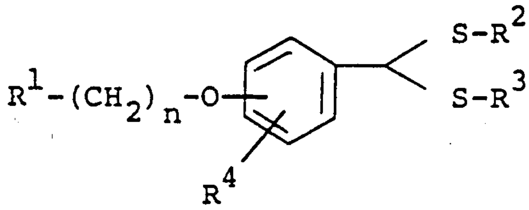

(I) or

reacting a compound of the following formula or its salt :

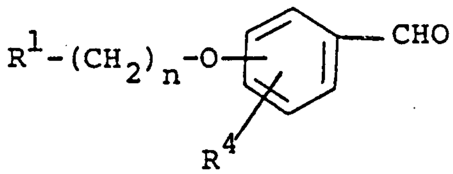

(IV)

with a compound of the following formula or its salt :

$R^2$-SH (V)

to give a compound of the following formula or its salt :

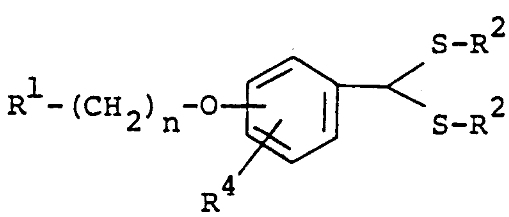

(Ia) or

reacting a compound of the following formula or its salt :

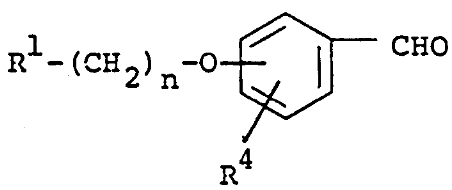

(IV)

with a compound of the following formula or its salt :

SH-$(CH_2)_3$-SH (VI)

to give a compound of the following formula or its salt :

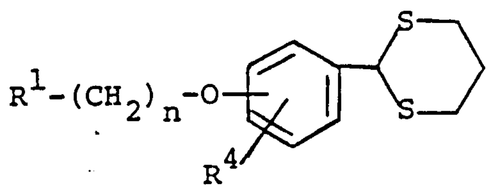

(Ib) or

reacting a compound of the following formula or its salt :

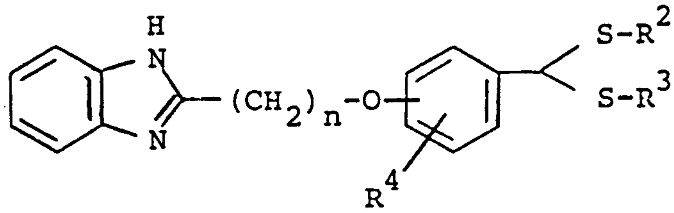

(Ic)

with ($C_1$-$C_6$)alkyl-alkylating agents to give a compound of the following formula or its salt:

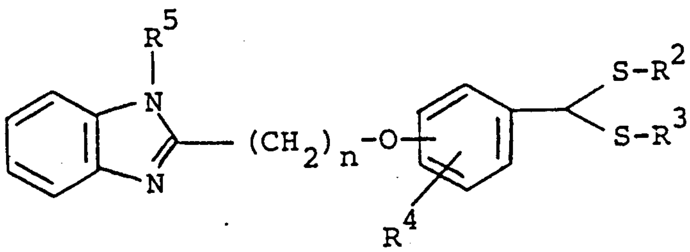

(Id)

wherein in the above formulae $R^1$, $R^2$, $R^3$, $R^4$ and n have the meaning as defined above, $R^5$ is ($C_1$-$C_6$)alkyl and Y is an acid residue selected from the group consisting of an inorganic acid residue of an inorganic acid selected from the group consisting of hydrochloric acid, hydrobromic acid, hydroiodic acid, sulfuric acid, of an organic acid selected from the group consisting of sulfonic acid, selected from methanesulfonic acid, benzenesulfonic acid and toluenesulfonic acid, and dimethylcarbamic acid.

The various processes according to which the novel dithioacetal compound (I) of the present invention can be prepared, can be illustrated by the following schemes:

Process 1

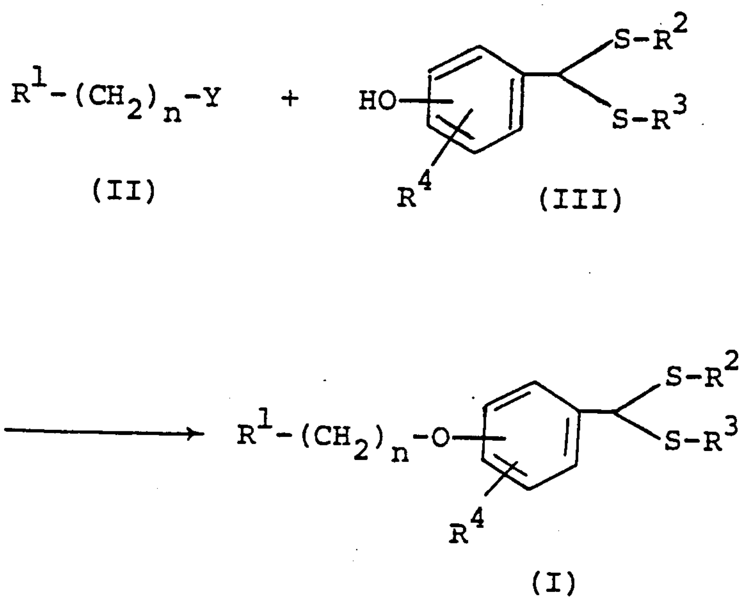

Process 2

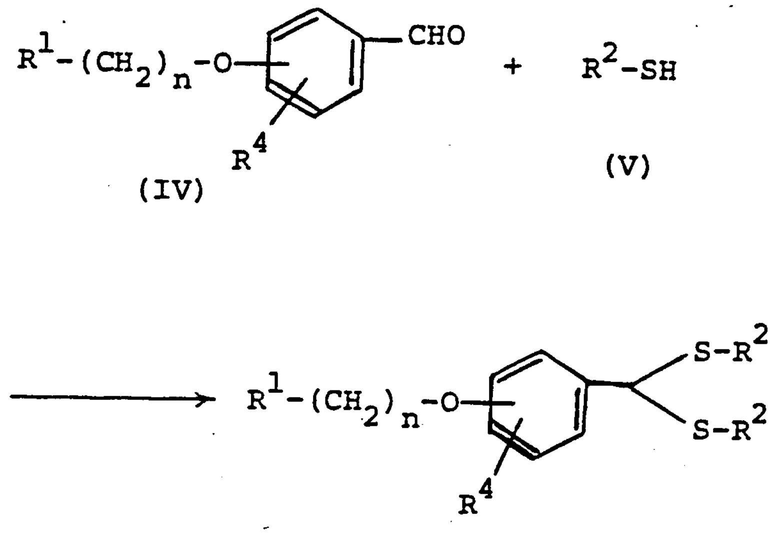

(IV) (V)

(Ia)

Process 3

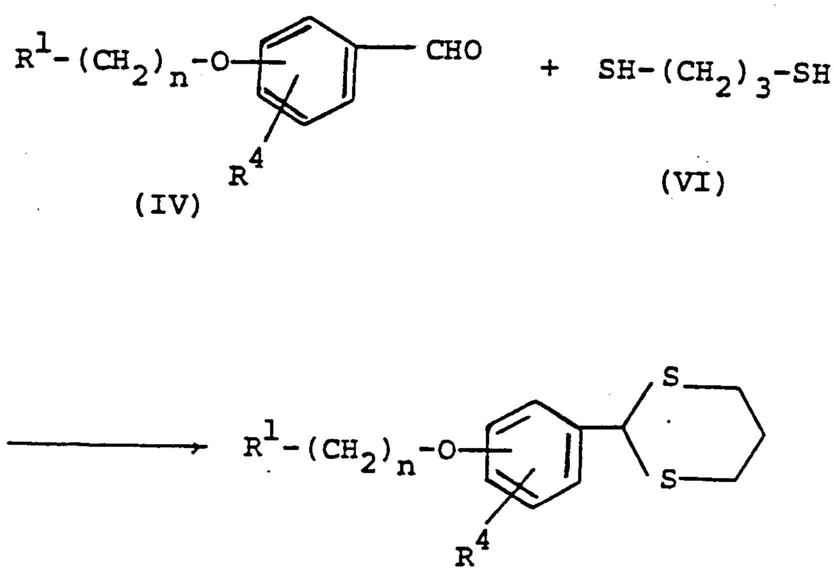

(IV) (VI)

(Ib)

Process 4

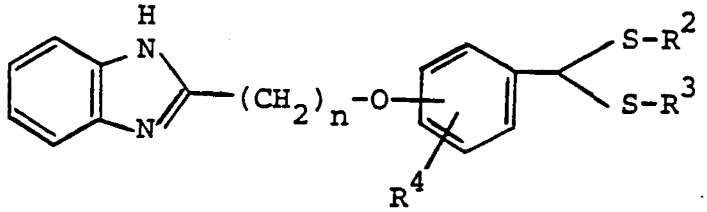

(Ic)

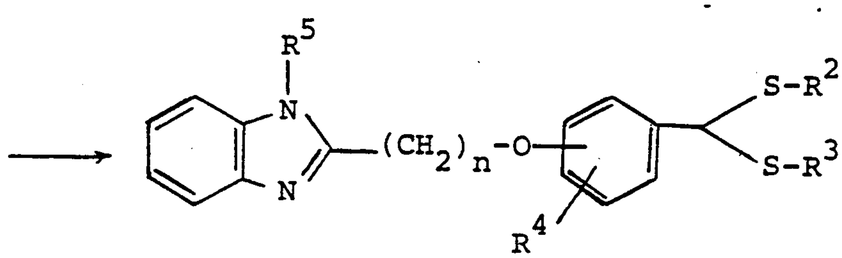

(Id)

In the above formulae, Y is an acid residue, $R^5$ is lower alkyl, and $R^1$, $R^2$, $R^3$, $R^4$ and n are as defined above.

Preferred embodiments of the process of the present invention as defined above are the following:

(a') a process as defined above, wherein
$R^1$ is heterocyclic group,
n is 1,
$R^2$ and $R^3$ are each($C_1$-$C_6$)alkyl, and
$R^4$ is hydrogen;

(b') a process as defined above, wherein the heterocyclic group is unsaturated benzene-fused 5 or 6-membered heterocyclic group containing one or two nitrogen atoms as defined above;

(c') a process as defined above wherein
$R^1$ is quinolyl,
n is 1,
$R^2$ and $R^3$ are each propyl, and
$R^4$ is hydrogen;

(d') a process as defined above wherein the heterocyclic group is represented the following formula:

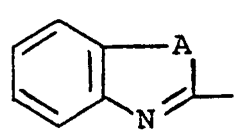

wherein A is

$$\overset{H}{-N-},$$

-O- or -S-; and

(e') a process as defined above wherein $R^1$ is benzothiazolyl, n is 1, $R^2$ and $R^3$ are each propyl, and $R^4$

is hydrogen.

According to a third aspect the present invention relates to a pharmaceutical composition which comprises as an active ingredient a compound of general formula (I) as defined above or its pharmaceutically acceptable salts.

Furthermore, it relates to an antiinflammatory agent or anti-allergic agent which comprises as an active ingredient a compound of general formula (I) as defined above or its pharmaceutically acceptable salt.

In addition, the present invention relates to a compound of general formula (I) or pharmaceutical acceptable salts thereof for use as a medicament and for use in treating or preventing inflammatory or allergic diseases.

Finally, the present invention relates to the use of a compound of general formula (I) and its pharmaceutically acceptable salt for the manufacture of a medicament for therapeutic treatment of inflammatory and allergic diseases.

Preferred pharmaceutically acceptable salts of the object compound (I) are conventional non-toxic salts and may include a salt with an acid such as a salt with an inorganic acid (e.g., hydrochloride, hydrobromide, sulfate and phosphate), or a salt with an organic carboxylic or sulfonic acid (e.g., formate, acetate, trifluoroacetate, maleate, tartrate, methanesulfonate, bensenesulfonate, and p-toluenesulfonate).

Preferred examples and illustrations of the various definitions, in the above descriptions, which the present invention includes within the scope thereof are explained in detail as follows.

The term "($C_1$-$C_6$)" is intended to mean 1 to 6 carbon atoms.

Preferred examples of "($C_1$-$C_6$)alkyl" and "($C_1$-$C_6$)alkyl moiety" may include a residue of straight and branched alkane having 1 to 6 carbon atom(s) such as methyl, ethyl, propyl, isopropyl, butyl, isobutyl, t-butyl, pentyl, isopentyl, neopentyl, hexyl, and isohexyl.

Preferred examples of "($C_1$-$C_6$)alkoxy" may include methoxy, ethoxy, propoxy, isopropoxy and butoxy.

The expressions "aryl" and "aryl moiety" include phenyl, totolyl, xylyl, and naphthyl.

The expression "heterocyclic group" includes a unsaturated 3-to 8-membered monocyclic heterocyclic group containing 1 to 4 nitrogen atoms such as pyrrolyl, pyrrolinyl, imidazolyl, pyrazolyl, pyridyl and its N-oxide, dihydropyridyl, pyrimidyl, pyrazinyl, pyridazinyl, triazolyl (e.g. 4H-1,2,4-triazolyl, 1H-1,2,3-triazolyl and 2H-1,2,3-triazolyl), and tetrazolyl (e.g. 1H-tetrazolyl and 2H-tetrazolyl), a 3- to 8-membered monocyclic heterocyclic group containing at least one sulfur atom and at least one nitrogen atom such as thiazolyl, isothiazolyl and thiadiazolyl, a polycyclic (e.g. bicyclic) heterocyclic group containing at least one nitrogen atom such as indolyl, isoindolyl, indolizinyl, benzimidazolyl, quinolyl, isoquinolyl, quinazolinyl, and imidazo-[1,2-a]pyridyl, a polycyclic (e.g. bicyclic) heterocyclic group containing at least one sulfur atom and at least one nitrogen atom such as benzothiazolyl, benzothiadiazolyl, a polycyclic (e.g. bicyclic) heterocyclic group containing at least one oxygen atom such as benzofuranyl, and isobenzofuranyl, a polycyclic (e.g. bicyclic) heterocyclic group containing at least one oxygen atom and at least one nitrogen atom such as benzoxazolyl and benzoxadiazolyl.

Preferred examples of "heterocyclic group" may include unsaturated benzene-fused 5-or 6-membered heterocyclic group containing one or two nitrogen atoms such as benzimidazolyl, quinolyl, isoquinolyl and quinazolinyl, and unsaturated benzene-fused 5-or 6-membered heterocyclic group containing one nitrogen atom and one sulfur or oxygen atom such as benzothiazolyl and benzoxazolyl.

More preferred examples of "heterocyclic group" may be represented by the following formula:

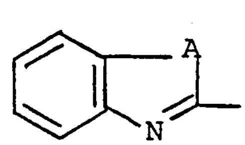

wherein A is

$$-\overset{H}{N}-,$$

-O- or -S-.

Preferred examples of "halogen" are fluorine, chlorine, bromine and iodine.

Preferred examples of "acid residue" may include an acid residue of an inorganic acid (e.g. hydrochloric acid, hydrobromic acid, hydroiodic acid, and sulfuric acid), an organic acid such as organic sulfonic acid

(e.g. methanesulfonic acid, benzenesulfonic acid, and toluenesulfonic acid), and an organic carbamic acid (e.g. dimethylcarbamic acid).

Processes for preparing the object compound (I) of this invention are explained in detail in the following.

Process 1

This process relates to one for preparing the compound (I) or its salt by reacting the compound (II) or its salt with the compound (III).

Suitable salts of the compounds (I) and (II) may include the same as those exemplified as pharmaceutically acceptable salts of the compound (I) hereinbefore.

The reaction may be preferably conducted in the presence of a base. Suitable base may be an inorganic base such as alkali or alkaline earth metal hydroxide (e.g. sodium hydroxide, potassium hydroxide, and calcium hydroxide,), alkali metal bicarbonate (e.g. sodium bicarbonate, and potassium bicarbonate), alkali or alkaline earth metal carbonate (e.g. sodium carbonate, potassium carbonate, and calcium carbonate), alkali metal phosphate (e.g. sodium dihydrogenphosphate, potassium dihydrogenphosphate, disodium hydrogenphosphate, and dipotassium hydrogenphosphate) or an organic base such as alkali metal alkoxide (e.g. sodium methoxide, and potassium ethoxide), amines (e.g. triethylamine, pyridine, and lutidine).

The reaction is usually conducted in conventional manner. For example, the reaction is preferably conducted under cooling, at ambient temperature or under heating, and in conventional solvent which does not have an adverse influence on the reaction such as water, methanol, ethanol, propanol, N,N-dimethylformamide or an optional mixture thereof.

Process 2

This process relates to one for preparing the compound ($I^a$) or its salt by reacting the compound (IV) or its salt with the compound (V) or its salt.

Suitable salts of the compounds ($I^a$), (IV) and (V) may include the same as those exemplified as pharmaceutically acceptable salts of the compound (I).

This reaction may preferably be conducted in the presence of an acid or Lewis acid.

Suitable acids may include an organic acid (e.g. formic acid, acetic acid, and propionic acid) and an inorganic acid (e.g. hydrochloric acid, hydrobromic acid, and sulfuric acid) and suitable examples of Lewis acid are boron trihalide (e.g. boron trichloride, and boron trifluoride), titanium tetrahalide (e.g. titanium tetrachloride, and titanium tetrabromide), tin tetrahalide (e.g. tin tetrachloride, and tin tetrabromide,), aluminum halide (e.g. aluminum chloride, and aluminum bromide), trihaloacetic acid (e.g. trichloroacetic acid, and trifluoroacetic acid).

The reaction is usually conducted under cooling, at ambient temperature or under heating, and in conventional solvent which does not have an adverse influence on the reaction such as acetonitril, dioxane, benzene, hexane, chloroform, dichloromethane, and tetrahydrofuran.

Process 3

This process relates to one for preparing the compound ($I^b$) or its salt by reacting the compound (IV) or its salt with the compound (VI) or its salt.

Suitable salts of the compounds ($I^b$), (IV) and (VI) may include the same as those exemplified as pharmaceutically acceptable salts of the compound (I) hereinbefore.

This reaction is conducted in substantially the same manner as that of Process 2 and is to be referred thereto.

Process 4

This process relates to one for preparing the compound ($I^d$) or its salt by reacting the compound ($I^c$) or its salt with alkylating agents.

Suitable salts of the compounds ($I^c$) and ($I^d$) may include the same as those exemplified as pharmaceutically acceptable salts of the compound (I) hereinbefore.

Examples of alkylating agents include diazoalkanes (e.g., diazomethane, and diazoethane), dialkyl sulphates (e.g., dimethyl sulphate, diethyl sulphate, and dipropyl sulphate), alkyl halides (e.g., methyl iodide, ethyl iodide, and propyl iodide).

The reaction is usually conducted in a conventional solvent which does not have an adverse influence on the reaction, for example, water, acetone, methanol, ethanol, benzene, toluene, dioxan, dichloromethane, N,N-dimethylformamide, and dimethyl sulphoxide, under cooling, at ambient temperature or under heating.

The reaction is preferably conducted in the presence of a base, examples of which include those given in the description of the Process 1.

Pharmaceutically acceptable salts of the compound (I) can be prepared by a conventional method, i.e., by treating the compound (I) with an acid. Preferred examples of said acid are the same as those exemplified in the explanation of pharmaceutically acceptable salts of the compound (I).

The starting compounds (III) and (IV) and their salts are novel and can be prepared, for example, according to Preparations as illustrated hereinafter or a similar manner thereto.

The object compound, dithioacetal compounds (I) and pharmaceutically acceptable salts thereof of this invention have 5-lipoxygenase-inhibiting activity (inhibiting activity of SRS-A synthesis), and are useful as an anti-allergic agent or an antiinflammatory agent for human being and animals, and more particulary are useful for treatment of asthma, psoriasis, hepatitis, pancreatitis, arthritis, nephritis, inflammatory bowel disease, septic shock, arteriosclerosis, myocardial infarction, and cerebral vasospasm.

In order to illustrate the usefulness of the object compound (I), the pharmacological test data of the representative compounds of the object compound (I) are shown below.

Test : Inhibiting activity of SRS-A (Slow Reacting Substance of Anaphylaxis) synthesis in rat polymorpholeukocyte (PMN) of using the calcium ionophore

(1) Materials and Methods :

Preparation of PMN from rat

Male Spraque-Dawley rats weighing 250-300 g were anesthetized with ether and each was injected intraperitoneally with 20 ml of 0.1% glycogen (from Oyster). After 20 hours the rats were sacrificed and PMN were recovered in rinse of the peritoneal cavity with 10 ml Dulbeccos PBS (components in G/L : $CaCl_2$ 0.1, $KH_2PO_4$ 0.2, $MgCl_2 \cdot 6H_2O$ 0.1, NaCl 8.0, $Na_2HPO_4 \cdot 7H_2O$ 2.16; pH 7.4). These rinses were filtered through nylon wool filter and centrifuged for 5 min at 1000 xg.
The pellet was suspended in Dulbeccos PBS and centrifuged for 5 min at 1000 xg. The pellet was resuspended in Dulbeccos PBS and adjusted the cell concentration to $10^7$ cells/ml with Dulbeccos PBS.

PMN stimulation

Samples were dissolved in ethanol and dispersed in Dulbeccos PBS to give a concentration of $10^{-10}$ to $10^{-5}$ M. Antibiotic A23187; calcium ionophor (Dehring Diagnostics) (hereinafter referred to A23187) in DMSO (10mM) was diluted with Dulbeccos PBS to give the concentration of 1mM. Aliquots of the cell suspension (1x$10^7$ cells/ml, 0.98 ml) were equilibrated for 30 min at 37°C. Solution of sample (10 $\mu\ell$) was added and incubated for 15 min at 37°C before the addition of 10 $\mu\ell$ of A23187 solution. Thus the final incubation volume of 1 ml contained approximately 1x$10^7$ cells, $10^{-10}$ to $10^{-5}$ M samples and 10$\mu$M A23187. Incubation with A23187 were continued for 15 min at 37°C. The reactions were terminated by setting the assay tubes in ice bath to chill as rapidly as possible to 4°C. The test tubes were centrifuged at 1500 xg for 5 min at 4°C and decanted the supernatants into the tubes and kept cold prior to assaying.

Determination of immunoreactive LTC4 (i-LTC4)

The concentration of i-LTC4 in the cell-free supernatants from the incubations were determined by specific radioimmunoassay. The mean values of i-LTC4 (these incubations were carried out in duplicate) of each sample were calculated and the effect of samples on the synthesis of the leukotrienes was presented as a percentage of the value in the absence of samples.

(2) Results :

| Test Compound (Example No.) | $IC_{50}$ (M) |
|---|---|
| 1 | $3.0 \times 10^{-7}$ |
| 2 | $5.2 \times 10^{-7}$ |
| 3 | $3.9 \times 10^{-8}$ |
| 5 | $1.1 \times 10^{-7}$ |
| 6 | $1.3 \times 10^{-7}$ |
| 7 | $1.6 \times 10^{-7}$ |
| 8 | $3.3 \times 10^{-7}$ |
| 9 | $1.0 \times 10^{-7}$ |
| 10 | $1.1 \times 10^{-6}$ |
| 11 | $1.4 \times 10^{-6}$ |
| 16 | $1.1 \times 10^{-7}$ |
| 22 | $8.5 \times 10^{-7}$ |

Pharmaceutical compositions of this invention can be used in a conventional pharmaceutical forms such as powders, fine granules, granules, tablets, dragee, microcapsules, capsules, suppository, solution, suspension, emulsion, and syrups. If desired, diluents or disintegrators (e.g. sucrose, lactose, starch, crystalline cellulose, low-substituted hydroxypropyl cellulose, and synthetic aluminum silicate,), binding agents (e.g. cellulose, methylcellulose, hydroxypropylcellulose, hydroxypropylmethylcellulose, polypropylpyrrolidone, polyvinylpyrrolidone, gelatin, gum arabic, and polyethyleneglycol), coloring agents, sweeting agents, lubricant (e.g. magnesium stearate,), may be dispensed with said composition.

The dosage of said composition of this invention depends on the patient's age, body weight, and condition, and it is generally administered by the oral route at the daily dose level of 100 mg to 10 g as the object compound (I) or its pharmaceutically acceptable salt, preferably 1 g to 5g on the same basis, at the interval of 1 to 3 times a day. Typical unit doses may be 50 mg, 100 mg, 200 mg, 500 mg, and 1 g, although these are only examples and not limitative, of course.

The following Preparations and Examples are given for the purpose of illustrating this invention.

Preparation 1

A mixture of 3-hydroxybenzaldehyde (6.71 g), 2-(chloromethyl)quinoline (8.88 g) and potassium carbonate (8.28 g) in N,N-dimethylformamide (25 ml) was stirred at 60°C for 3 hours. The mixture was allowed to cool and concentrated in vacuo. The residue was treated with water. The separated oil was extracted with ethyl acetate. The organic layer was washed with brine, dried, and evaporated to give an oil, which was crystallized from n-hexane to yield 2-[[3-(formyl)phenoxy]methyl]quinoline (12.5 g).

mp : 62-63°C

NMR ($CDCl_3$, $\delta$) : 5.45 (2H, s), 7.30 (1H, d, J=8Hz), 7.45 (1H, t, J=8Hz), 7.50 (1H, s), 7.52-7.62 (2H, m), 7.65 (1H, d, J=8Hz), 7.76 (1H, t, J=8Hz), 7.85 (1H, d, J=8Hz), 8.10 (1H, d, J=8Hz), 8.21 (1H, d, J=8Hz), 10.00 (1H, s)

Following compounds were prepared according to a similar manner to that of Preparation 1.

Preparation 2

2-[[4-(Formyl)phenoxy]methyl]quinoline

mp : 88-89°C

NMR ($CDCl_3$, $\delta$) : 5.47 (2H, s), 7.12 (2H, d, J=8Hz), 7.56 (1H, t, J=8Hz), 7.64 (1H, d, J=8Hz), 7.70-7.90 (4H, m), 8.10 (1H, d, J=8Hz), 8.21 (1H, d, J=8Hz), 9.88 (1H, s)

Preparation 3

2-[[2-(Formyl)phenoxy]methyl]quinoline

mp : 100-104°C

NMR ($CDCl_3$, $\delta$) : 5.51 (2H, s), 7.00-7.20 (2H, m), 7.50-7.65 (2H, m), 7.71 (1H, d, J=8Hz), 7.79 (1H, t, J=8Hz), 7.88 (1H, d, J=8Hz), 7.91 (1H, d, J=8Hz), 8.11 (1H, d, J=8Hz), 8.26 (1H, d, J=8Hz), 10.70 (1H, s)

Preparation 4

3-[Bis(propylthio)methyl]-1-isopropoxybenzene (oil)

NMR ($CDCl_3$, δ) : 0.95 (6H, t, J = 7.4Hz), 1.34 (6H, d, J = 6.1Hz), 1.43-1.67 (4H, m), 2.42-2.64 (4H, m), 4.50-4.62 (1H, m), 4.82 (1H, s), 6.78 (1H, dd, J = 2.5, 7.8Hz), 6.95-7.00 (2H, m), 7.21 (1H, t, J = 7.8Hz)

IR (Nujol) : 1595, 1255, 985 $cm^{-1}$

Preparation 5

2-[[(5-Formyl-2-methoxy)phenoxy]methyl]quinoline

mp : 119-120 °C

NMR ($CDCl_3$, δ) : 4.00 (3H, s), 5.51 (2H, s), 7.04 (1H, d, J = 8Hz), 7.45-7.63 (3H, m), 7.69 (1H, d, J = 8Hz), 7.76 (1H, t, J = 8Hz), 7.83 (1H, d, J = 8Hz), 8.11 (1H, d, J = 8Hz), 8.20 (1H, d, J = 8Hz), 9.80 (1H, s)

IR (Nujol) : 1665, 1595, 1580, 1505, 1435 $cm^{-1}$

Example 1

A mixture of [3-bis(propylthio)methyl]phenol (900 mg), 2-(chloromethyl)benzothiazole (643 mg) and potassium carbonate (532 mg) in N,N-dimethylformamide (5 ml) was stirred at 60 °C for 3 hours. To the mixture was added water and the separated oil was extracted with ethyl acetate. The organic layer was washed with water, dried, and evaporated under reduced pressure to give an yellow oil, which was crystallized from n-hexane to yield 2-[[[3-bis(propylthio)methyl]phenoxy]methyl]benzothiazole (936 mg).

mp : 49-51 °C

NMR ($CDCl_3$, δ) : 0.93 (6H, t, J = 7.4Hz), 1.46-1.64 (4H, m), 2.39-2.61 (4H, m), 4.84 (1H, s), 5.50 (2H, s), 6.93-6.97 (1H, m), 7.08 (1H, d, J = 7.7Hz), 7.16-7.51 (4H, m), 7.90 (1H, dd, J = 1.3Hz and 7.4Hz), 8.04 (1H, d, J = 7.4Hz)

Example 2

2-[[[3-Bis(propylthio)methyl]phenoxy]methyl]pyridine hydrochloride was prepared according to a similar manner to that of Example 1 and by treating the object compound, 2-[[[3-bis(propylthio)methyl]phenoxy]-methyl]pyridine with dry hydrogen chloride in ethyl ether.

mp : 74-76 °C

NMR ($CD_3OD$, δ) : 0.94 (6H, t, J = 7.3Hz), 1.51-1.62 (4H, m), 2.45-2.61 (4H, m), 4.97 (1H, s), 5.56 (2H, s), 7.07 (1H, dd, J = 2.4Hz and 7.8Hz), 7.72 (1H, d, J = 7.7Hz), 7.26-7.37 (2H, m), 8.09 (1H, t, J = 8.4Hz and 5.8Hz), 8.2 (1H, d, J = 8.0Hz), 8.68 (1H, t, J = 8.0Hz and 8.4Hz), 8.88 (1H, d, J = 5.8Hz)

Example 3

To a cooled mixture of 2-[[3-(formyl)phenoxy]methyl]quinoline (5.26 g) and 1-propanthiol (4 ml) in dry acetonitrile (30 ml) in an ice bath was added dropwise boron trifluoride etherate (3.7 ml) in a few minutes. The mixture was stirred at 0 °C for one hour and then dry ethyl ether (150 ml) was added thereto. The separated solid was collected by filtration, washed with ethyl ether, and dissolved in a mixture of ethyl ether and aqueous 1N-sodium hydroxide solution. The organic layer was washed with brine, dried, and evaporated under reduced pressure. The residue was dissolved in dry ethyl ether (50 ml) and treated with dry hydrogen chloride to yield 2-[[[3-bis(propylthio)methyl]phenoxy]methyl]quinoline hydrochloride (800 mg)

mp : 127-129 °C

NMR (DMSO-$d_6$, δ) : 0.86 (6H, t, J = 6Hz), 1.45 (4H, m), 2.43 (4H, m), 5.07 (1H, s), 5.58 (2H, s), 7.02 (1H, d, J = 8Hz), 7.07 (1H, d, J = 8Hz), 7.18 (1H, s), 7.30 (1H, t, J = 8Hz), 7.78 (1H, t, J = 8Hz), 7.92 (1H, d, J = 8Hz), 7.99 (1H, t, J = 8Hz), 8.19 (1H, d, J = 8Hz), 8.28 (1H, d, J = 8Hz), 8.80 (1H, d, J = 8Hz)

Following compounds were prepared according to a similar manner to that of Example 3.

Example 4

2-[[[2-Bis(propylthio)methyl]phenoxy]methyl]quinoline hydrochloride

mp : 127-129 °C

NMR (DMSO-$d_6$, δ) : 0.83 (6H, t, J = 6Hz), 1.50 (4H, m), 2.51 (4H, m), 5.52 (1H, s), 5.58 (2H, s), 7.02 (1H, t, J = 8Hz), 7.14 (1H, d, J = 8Hz), 7.37 (1H, t, J = 8Hz), 7.55 (1H, d, J = 8Hz), 7.75 (1H, t, J = 8Hz), 7.89 (1H, d, J = 8Hz), 7.94 (1H, t, J = 8Hz), 8.15 (1H, d, J = 8Hz), 8.21 (1H, d, J = 8Hz), 8.74 (1H, d, J = 8Hz)

Example 5

2-[[[4-Bis(propylthio)methyl]phenoxy]methyl]quinoline hydrochloride

mp : 120-121 °C

NMR (DMSO-$d_6$, δ) : 0.88 (6H, t, J = 6Hz), 1.50 (4H, m), 2.48 (4H, m), 5.05 (1H, s), 5.51 (2H, s), 7.06 (2H, d, J = 8Hz), 7.39 (2H, d, J = 8Hz), 7.75 (1H, t, J = 8Hz), 7.87 (1H, d, J = 8Hz), 7.94 (1H, t, J = 8Hz), 8.15 (1H, d, J = 8Hz), 8.22 (1H, d, J = 8Hz), 8.13 (1H, d, J = 8Hz)

Example 6

2-[[[3-Bis(ethylthio)methyl]phenoxy]methyl]quinoline hydrochloride

mp : 114-115 °C

NMR ($CD_3OD$, δ) : 1.48 (6H, t, J = 7Hz), 2.43-2.60 (4H, m), 5.00 (1H, s), 5.65 (2H, s), 7.02 (1H, d, J = 8Hz), 7.11 (1H, d, J = 8Hz), 7.27 (1H, s), 7.31 (1H, t, J = 8Hz), 7.90 (1H, t, J = 8Hz), 8.08 (1H, d, J = 8Hz), 8.10 (1H, t, J = 8Hz), 8.27 (1H, d, J = 8Hz), 8.31 (1H, d, J = 8Hz), 9.01 (1H, d, J = 8Hz)

Example 7

2-[[[3-Bis(butylthio)methyl]phenoxy]methyl]quinoline hydrochloride

mp : 79-80 °C

NMR ($CD_3OD$, δ) : 0.86 (6H, t, J = 7Hz), 1.24-1.57 (8H, m), 2.45-2.60 (4H, m), 4.96 (1H, s), 5.74 (2H, s), 7.09 (1H, d, J = 8Hz), 7.13 (1H, d, J = 8Hz), 7.29 (1H, s), 7.33 (1H, t, J = 8Hz), 7.98 (1H, t, J = 8Hz), 8.18 (1H, d, J = 8Hz), 8.20 (1H, t, J = 8Hz), 8.35 (1H, d, J = 8Hz), 8.39 (1H, d, J = 8Hz), 9.17 (1H, d, J = 8Hz)

Example 8

2-[[[3-Bis(pentylthio)methyl]phenoxy]methyl]quinoline hydrochloride

mp : 89-90 °C

NMR (DMSO-$d_6$, δ) : 0.82 (6H, t, J = 6Hz), 1.10-1.35 (8H, m), 1.53 (4H, m), 2.43 (4H, m), 5.07 (1H, s), 5.53 (2H, s), 7.00 (1H, d, J = 8Hz), 7.05 (1H, d, J = 8Hz), 7.16 (1H, s), 7.30 (1H, t, J = 8Hz), 7.75 (1H, t, J = 8Hz), 7.87 (1H, d, J = 8Hz), 7.94 (1H, t, J = 8Hz), 8.15 (1H, d, J = 8Hz), 8.24 (1H, d, J = 8Hz), 8.73 (1H, d, J = 8Hz)

Example 9

2-[[[3-Bis(isopropylthio)methyl]phenoxy]methyl]quinoline hydrochloride

mp : 152-154 °C

NMR ($CD_3OD$, δ) : 1.16 (6H, d, J = 7Hz), 1.21 (6H, d, J = 7Hz), 2.82-2.96 (2H, m), 5.03 (1H, s), 5.72 (2H, s), 7.10 (1H, d, J = 8Hz), 7.15 (1H, d, J = 8Hz), 7.31 (1H, s), 7.33 (1H, t, J = 8Hz), 7.98 (1H, t, J = 8Hz), 8.17 (1H, d, J = 8Hz), 8.19 (1H, t, J = 8Hz), 8.34 (1H, d, J = 8Hz), 8.37 (1H, d, J = 8Hz), 9.16 (1H, d, J = 8Hz)

Example 10

2-[[[3-Bis(phenylthio)methyl]phenoxy]methyl]quinoline hydrochloride

mp : 143-145 °C

NMR (DMSO-$d_6$, δ) : 5.51 (2H, s), 6.11 (1H, s), 6.99 (1H, d, J = 8Hz), 7.13 (1H, d, J = 8Hz), 7.15-7.40 (12H, m), 7.77 (1H, t, J = 8Hz), 7.83 (1H, d, J = 8Hz), 7.96 (1H, t, J = 8Hz), 8.17

(1H, d, J=8Hz), 8.25 (1H, d, J=8Hz), 8.75 (1H, d, J=8Hz)

Example 11

2-[[[3-Bis(benzylthio)methyl]phenoxy]methyl]quinoline

mp : 90-91 °C

NMR ($CDCl_3$, $\delta$) : 3.56 (2H, d, J=14Hz), 3.78 (2H, d, J=14Hz), 4.47 (1H, s), 5.40 (2H, s), 6.90-7.00 (2H, m), 7.05-7.35 (12H, m), 7.57 (1H, t, J=8Hz), 7.71 (1H, d, J=8Hz), 7.77 (1H, t, J=8Hz), 7.86 (1H, d, J=8Hz), 8.12 (1H, d, J=8Hz), 8.23 (1H, d, J=8Hz)

Following compounds were prepared according to a similar manner to that of Example 1.

Example 12

2-[[[3-Bis(propylthio)methyl]phenoxy]methyl]-5-methoxybenzothiazole (oil)

NMR ($CDCl_3$, $\delta$) : 0.94 (6H, t, J=7Hz), 1.56 (4H, m), 2.51 (4H, m), 3.91 (3H, s), 4.83 (1H, s), 5.48 (2H, s), 6.95 (1H, d, J=8Hz), 7.06 (1H, d, J=8Hz), 7.08 (1H, d, J=8Hz), 7.17 (1H, s), 7.27 (1H t, J=8Hz), 7.52 (1H, s), 7.73 (1H, d, J=8Hz)

IR (Nujol) : 1595, 1575, 1480, 1320, 1255, 1150 $cm^{-1}$

Example 13

2-[[[3-Bis(propylthio)methyl]phenoxy]methyl]-5-chlorobenzothiazole

mp : 86-88 °C

NMR ($CDCl_3$, $\delta$) : 0.96 (6H, t, J=7.4Hz), 1.49-1.67 (4H, m), 2.45-2.61 (4H, m), 4.86 (1H, s), 5.50 (2H, s), 6.92-8.04 (7H, m)

Example 14

2-[[[3-Bis(propylthio)methyl]phenoxy]methyl]benzoxazole (oil)

NMR ($CDCl_3$, $\delta$) : 0.96 (6H, t, J=7.3Hz), 1.49-1.67 (4H, m), 2.41-2.60 (4H, m); 4.86 (1H, s), 5.36 (2H, s), 6.99 (1H, dd, J=2.5, 7.8Hz), 7.11 (1H, d, J=7.8Hz), 7.21 (1H, d, J=2.5Hz), 7.29 (1H, t, J=7.8Hz), 7.37-7.44 (2H, m), 7.57-7.61 (1H, m), 7.77-7.81 (1H, m)

IR (Nujol) : 1595, 1580, 1260, 1145 $cm^{-1}$

Example 15

2-[[[3-Bis(propylthio)methyl]phenoxy]methyl]quinazoline (oil)

NMR ($CDCl_3$, $\delta$) : 0.93 (6H, t, J=7.1Hz), 1.53 (4H, m), 2.47 (4H, m), 4.82 (1H, s), 5.51 (2H, s), 6.95-7.08 (2H, m), 7.21 (1H, s), 7.27 (1H, d, J=6.5Hz), 7.68 (1H, m), 8.00-8.10 (2H, m) , 8.09 (1H, m), 9.43 (1H, s)

IR ($CHCl_3$) : 2975, 2860, 1620, 1580, 1490, 1385, 1260, 1150 $cm^{-1}$

Example 16

2-[[[3-Bis(isopropylthio)methyl]phenoxy]methyl]benzothiazole

mp : 109-110 °C

NMR ($CDCl_3$, $\delta$) : 1.19 (6H, d, J=6.0Hz), 1.23 (6H, d, J=6.0Hz), 2.92 (2H, m), 4.91 (1H, s), 5.50 (2H, s), 6.94 (1H, d, J=7.7Hz), 7.12 (1H, d, J=7.7Hz), 7.21-7.31 (2H, m), 7.37-7.54 (2H, m), 7.91 (1H, d, J=6.7Hz), 8.04 (1H, d, J=6.7Hz)

IR (Nujol) : 2925, 2850, 1605, 1475, 1465, 1270, 760 $cm^{-1}$

Example 17

2-[[[3-Bis(propylthio)methyl]phenoxy]methyl]naphthalene (oil)

NMR ($CDCl_3$, $\delta$) : 0.99 (6H, t, J=7.3Hz), 1.53-1.71 (4H, m), 2.46-2.68 (4H, m), 4.88 (1H, s), 5.53 (2H, s), 6.99 (1H, d, J=8.2Hz), 7.09 (1H, d, J=7.6Hz), 7.22-7.67 (6H, m), 7.88-7.96 (2H, m), 8.10 (1H, d, J=9.4Hz)

IR (Nujol) : 1595, 1585, 1240, 1045 $cm^{-1}$

Example 18

2-[[[3-Bis(propylthio)methyl]phenoxy]methyl]-5-bromo-4-phenylthiazole

mp : 41-42 ° C

NMR ($CDCl_3$, δ) : 0.99 (6H, t, J = 7Hz), 1.47-1.70 (4H, m), 2.40-2.67 (4H, m), 4.85 (1H, s), 5.36 (2H, s), 6.91 (1H, dd, J = 2, 8Hz), 7.07-7.55 (7H, m), 7.94 (1H, d, J = 8Hz)

Example 19

A mixture of [3-bis(propylthio)methyl]phenol (180 mg), 2-chloromethylimidazo[1,2-a]pyridine (117 mg) and potassium carbonate (107 mg) in N,N-dimethylformamide (1 ml) was stirred at 80 ° C for 40 minutes. The reaction mixture was allowed to cool to ambient temperature, followed by addition of water. The separated oil was extracted with ethyl acetate. The organic layer was dried and concentrated in vacuo to give a brown oil (230 mg) which was purified by columnchromatography on silica gel, eluting with 1 % methanol in methylene chloride. The resulting colorless oil dissolved in a little amount of ethyl ether was treated with hydrogen chloride in ethyl acetate to give 2-[[[3-bis(propylthio)methyl]phenoxy]methyl]imidazo-[1,2-a]pyridine hydrochloride (130 mg).

mp : 75-80 ° C

NMR ($CD_3OD$, δ) : 0.92 (6H, t, J = 7Hz), 1.54 (4H, m), 2.51 (4H, m), 4.95 (1H, s), 5.41 (2H, s), 7.01 (1H, d, J = 8Hz), 7.09 (1H, d, J = 8Hz), 7.20 (1H, d, J = 8Hz), 7.29 (1H, t, J = 8Hz), 7.49 (1H, t, J = 7Hz), 7.85-8.05 (2H, m), 8.31 (1H, s), 8.80 (1H, d, J = 7Hz)

IR (Nujol) : 3470, 3400, 3100, 1655, 1600, 1580, 1525, 1480, 1255 $cm^{-1}$

Following compounds were prepared according to a similar manner to that of Example 19.

Example 20

2-[[3-Bis(propylthio)methyl]phenoxy]quinoline hydrochloride

mp : 108-110 ° C

NMR ($DMSO-d_6$, δ) : 0.89 (6H, t, J = 6Hz), 1.52 (4H, m), 2.53 (4H, m), 5.18 (1H, s), 7.17 (1H, d, J = 8Hz), 7.25-7.75 (7H, m), 7.96 (1H, d, J = 8Hz), 8.43 (1H, d, J = 8Hz)

IR (Nujol) : 1640, 1595, 1390, 1305, 1280, 1250, 1245, 755 $cm^{-1}$

Example 21

3-[Bis(propylthio)methyl]-1-(2-N,N-dimethylaminoethoxy) benzene hydrochloride

mp : 94-96 ° C

NMR ($CDCl_3$, δ) : 0.93 (6H, t, J = 7.3Hz), 1.47-1.65 (4H, m), 2.40-2.63 (4H, m), 2.97 (6H, s), 3.59 (2H, t, J = 4.8Hz), 4.35 (2H, t, J = 4.8Hz), 4.94 (1H, s), 6.93 (1H, dd, J = 2.5, 7.8Hz), 7.08 (1H, t, J = 7.8Hz), 7.15 (1H, d, J = 2.5Hz), 7.27 (1H, t, J = 7.8Hz)

IR (Nujol) : 1590, 1260, 1150, 1025 $cm^{-1}$

Example 22

2-[[[3-Bis(propylthio)methyl]phenoxy]methyl]quinazoline hydrochloride was prepared by treating 2-[[[3-bis(propylthio)methyl]phenoxy]methyl]quinazoline with hydrogen chloride in ethyl ether.

mp : 104-105 ° C (decomp.)

NMR ($CD_3OD$, δ) : 0.92 (3H, t, J = 6.2Hz), 0.94 (3H, t, J = 6.2Hz), 1.41-1.65 (4H, m), 2.73-2.63 (4H, m), 4.98 (1H, s), 5.31 (2H, s), 6.21 (1H, s), 7.06 (1H, dd, J = 2.0, 8.7Hz), 7.16 (1H, d, J = 8.7Hz), 7.28-7.65 (6H, m)

IR (Nujol) : 2925, 2850, 1650, 1585, 1465, 1320, 1285, 750 $cm^{-1}$

Following compounds were prepared according to a similar manner to that of Example 3.

Example 23

2-[[[5-Bis(propylthio)methyl]-2-methoxyphenoxy]methyl]quinoline hydrochloride

mp : 119-120 ° C

NMR ($DMSO-d_6$, δ) : 0.79 (6H, t, J = 7Hz), 1.39 (4H, m), 2.35 (4H, m), 3.82 (3H, s), 5.00 (1H, s), 5.55 (2H, s), 7.01 (2H, s), 7.19 (1H, s), 7.80 (1H, t, J = 8Hz), 7.90 (1H, d, J = 8Hz),

| | |
|---|---|
| | 7.99 (1H, t, J = 8Hz), 8.19 (1H, d, J = 8Hz), 8.28 (1H, d, J = 8Hz), 8.80 (1H, d, J = 8Hz) |
| IR (Nujol) : | 2250, 1975, 1595, 1505, 1410 $cm^{-1}$ |

Example 24

2-[[[3-Bis(propylthio)methyl]phenoxy]methyl]benzimidazole hydrochloride

| | |
|---|---|
| mp : | 130-133 ° C |
| NMR (DMSO-$d_6$, δ) : | 0.85 (6H, t, J = 7Hz), 1.48 (4H, m), 2.46 (4H, m), 5.12 (1H, s), 5.67 (2H, s), 7.06-7.85 (8H, m) |
| IR (Nujol) : | 2750-2300, 1605, 1265, 900 $cm^{-1}$ |

Example 25

To a cooled mixture of 2-[[3-(formyl)phenoxy]methyl]quinoline (1.05 g) and 1,3-propanedithiol (0.98 ml) in dry acetonitrile (10 ml) in an ice bath was added dropwise 2N-hydrogen chloride in ethyl acetate (4.8 ml). The mixture was stirred at 0 ° C for 3 hours and then dry ethyl ether (100 ml) was added thereto. The supernatant was discarded and the syrupy residue was washed with dry ethyl ether twice. The residue was dissolved in methanol (30 ml) and then aqueous 1N-sodium hydroxide solution (10 ml) was added dropwise thereto at 0 ° C. To the mixture were added methylene chloride (100 ml) and water (80 ml) and then the separated organic layer was dried and evaporated in vacuo to give a syrup (1.60 g), which was purified by column chromatography on silica gel (elution by methylene chloride) to give the objective product (1.04 g) as a colorless syrup. The syrup (910 mg) was crystallized from ethyl ether to yield 2-[[3-(1,3-dithian-2-yl)-phenoxy]methyl]quinoline (740 mg) as white powders.

| | |
|---|---|
| mp : | 92-93 ° C |
| NMR ($CDCl_3$, δ) : | 1.95 (1H, m), 2.17 (1H, m), 2.88-2.93 (2H, m), 3.01-3.09 (2H, m), 5.13 (1H, s), 5.38 (2H, s), 6.95 (1H, d, J = 8Hz), 7.08 (1H, d, J = 8Hz), 7.20 (1H, s), 7.25 (1H, t, J = 8Hz), 7.55 (1H, t, J = 8Hz), 7.68 (1H, d, J = 8Hz), 7.74 (1H, t, J = 8Hz), 7.83 (1H, d, J = 8Hz), 8.08 (1H, d, J = 8Hz), 8.20 (1H, d, J = 8Hz) |
| IR (Nujol) : | 1596, 1273, 1156 $cm^{-1}$ |

Example 26

A mixture of 2-[[[3-bis(propylthio)methyl]phenoxy]methyl]benzimidazole hydrochloride (480 mg), methyl iodide (356 mg) and potassium carbonate (169 mg) in N,N-dimethylformamide (6 ml) was stirred for 3 hours at ambient temperature and then for 1.5 hours at 40 ° C. The mixture was cooled to ambient temperature and poured into a mixture of ice-water and ethyl ether. The organic layer was separated and extracted with 1N hydrochloric acid. After adjustment to pH 8 with sodium bicarbonate, the aqueous layer was extracted with methylene chloride. The organic layer was dried and evaporated in vacuo to give an oily residue (190 mg), which was purified by preparative T.L.C., developed with a mixture of benzene and acetone (8.5 : 1.5 (V/V)), to yield an oily product (100 mg). To a solution of the product in ethyl acetate (2 ml) was added 2N-hydrogen chloride in ethyl acetate (0.5 ml) to give white precipitates, which were collected by filtration and washed with ethyl acetate to yield 2-[[[3-bis(propylthio)methyl]phenoxy]methyl]-1-methylbenzimidazole hydrochloride (85 mg) as white crystals.

| | |
|---|---|
| mp : | 148-149 ° C |
| NMR (DMSO-$d_6$, δ) : | 0.87 (6H, t, J = 6Hz), 1.50 (4H, m), 2.47 (4H, m), 4.05 (3H, s), 5.12 (1H, s), 5.74 (2H, s), 7.11-8.00 (8H, m) |

Example 27

3-[Bis(propylthio)methyl]-1-isopropoxybenzene (oil) was prepared according to a similar manner that of Example 1.

| | |
|---|---|
| NMR ($CDCl_3$, δ) : | 0.95 (6H, t, J = 7.4Hz), 1.34 (6H, d, J = 6.1Hz), 1.43-1.67 (4H, m), 2.42-2.64 (4H, m), 4.50-4.62 (1H, m), 4.82 (1H, s), 6.78 (1H, dd, J = 2.5, 7.8Hz), 6.95-7.00 (2H, m), 7.21 (1H, t, J = 7.8Hz) |
| IR (Nujol) : | 1595, 1255, 985 $cm^{-1}$ |

**Claims**

**Claims for the following Contracting States : AT, BE, CH, DE, FR, GB, IT, LI, LU, NL, SE**

1. A dithioacetal compound of the formula :

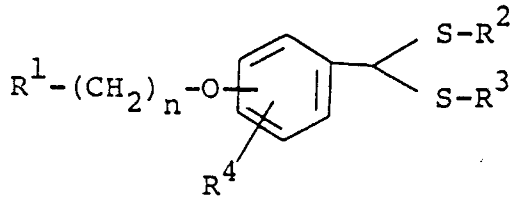

wherein

$R^1$ is $(C_1$-$C_6)$alkyl, di-$(C_1$-$C_6)$alkylamino, aryl selected from the group consisting of phenyl, tolyl, xylyl or naphthyl, or heterocyclic group which may have one or more substituents selected from halogen, $(C_1$-$C_6)$alkyl, $(C_1$-$C_6)$alkoxy and phenyl, the heterocyclic group being selected from the group consisting of an unsaturated 3- to 8-membered monocyclic heterocyclic group containing 1 to 4 nitrogen atoms selected from the group consisting of pyrrolyl, pyrrolinyl, imidazolyl, pyrazolyl, pyridyl and its N-oxide, dihydropyridyl, pyrimidyl, pyrazinyl, pyridazinyl, triazolyl, a 3- to 8-membered monocyclic heterocyclic group containing at least one sulfur atom and at least one nitrogen atom selected from the group consisting of thiazolyl, isothiazolyl, thiadiazolyl, a polycyclic heterocyclic group containing at least one nitrogen atom selected from the group consisting of indolyl, isoindolyl, indolizinyl, benzimidazolyl, quinolyl, isoquinolyl, quinazolinyl, imidazo(1,2-a)pyridyl, a polycyclic heterocyclic group containing at least one sulfur atom and at least one nitrogen atom selected from the group consisting of benzothiazolyl, benzothiadiazolyl, a polycyclic heterocyclic group containing at least one oxygen atom selected from the group consisting of benzofuranyl, isobenzofuranyl, a polycyclic heterocyclic group containing at least one oxygen atom and at least one nitrogen atom selected from the group consisting of benzoxazolyl, benzoxadiazolyl,

$R^2$ and $R^3$ are each $(C_1$-$C_6)$alkyl, aryl or ar-$(C_1$-$C_6)$alkyl whereby aryl and the aryl moiety are selected from the group consisting of phenyl, tolyl, xylyl or naphthyl, or $R^2$ and $R^3$ are together to form 1,3-dithian ring,

$R^4$ is hydrogen or $(C_1$-$C_6)$alkoxy,

and

n is 0 or an integer of 1 to 4, and pharmaceutical acceptable salts thereof.

2. A compound according to claim 1,
wherein
$R^1$ is a heterocyclic group as defined in claim 1
n is 1,
$R^2$ and $R^3$ are each $(C_1$-$C_6)$alkyl, and
$R^4$ is hydrogen.

3. A compound according to claim 1, wherein the compound is represented by the following formula:

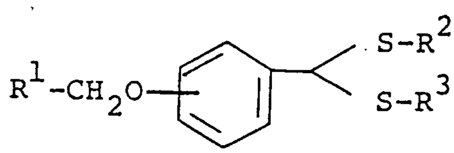

wherein $R^1$ is a heterocyclic group as defined in claim 1 which may have one or more substituents selected from halogen, $C_1$-$C_6)$alkyl, $(C_1$-$C_6)$alkoxy and phenyl, and

$R^2$ and $R^3$ are each $(C_1$-$C_6)$alkyl.

4. A compound according to claim 1,
wherein the compound is represented by the following formula :

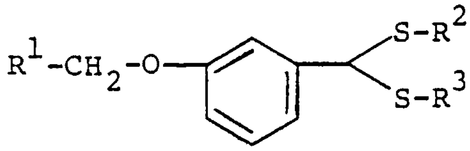

wherein
$R^1$ is unsaturated benzene-fused 5 or 6-membered heterocyclic group containing one or two nitrogen atoms as defined in claim 1, and
$R^2$ and $R^3$ are each ($C_1$-$C_6$) alkyl.

**5.** A compound according to claim 4,
wherein
$R^1$ is quinolyl, and
$R^2$ and $R^3$ are each propyl.

**6.** A compound according to claim 4,
wherein
$R^1$ is a heterocyclic group of the formula :

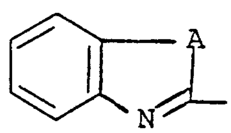

wherein
A is

$$\overset{H}{-N-},$$

-O- or -S- and
$R^2$ and $R^3$ are each ($C_1$-$C_6$)alkyl.

**7.** A compound according to claim 6,
wherein
$R^1$ is benzothiazolyl, and
$R^2$ and $R^3$ are each propyl.

**8.** A process for preparation of a compound of the following formula :

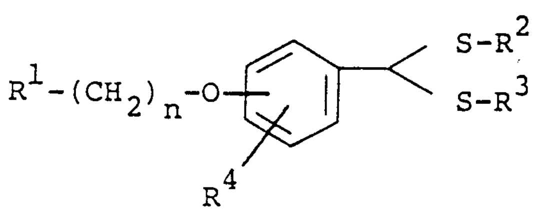

wherein
$R^1$ is ($C_1$-$C_6$)alkyl, di-($C_1$-$C_6$)alkylamino, aryl selected from the group consisting of phenyl, tolyl, xylyl or naphthyl, or heterocyclic group which may have one or more substituents selected from halogen, ($C_1$-$C_6$)alkyl, ($C_1$-$C_6$)alkoxy and phenyl, the heterocyclic group being selected from the group consisting of an unsaturated 3- to 8-membered monocyclic heterocyclic group containing 1 to 4 nitrogen atoms selected from the group consisting of pyrrolyl, pyrrolinyl, imidazolyl, pyrazolyl, pyridyl

and its N-oxide, dihydropyridyl, pyrimidyl, pyrazinyl, pyridazinyl, triazolyl, a 3- to 8-membered monocyclic heterocyclic group containing at least one sulfur atom and at least one nitrogen atom selected from the group consisting of thiazolyl, isothiazolyl, thiadiazolyl, a polycyclic heterocyclic group containing at least one nitrogen atom selected from the group consisting of indolyl, isoindolyl, indolizinyl, benzimidazolyl, quinolyl, isoquinolyl, quinazolinyl, imidazo(1,2-a)pyridyl, a polycyclic heterocyclic group containing at least one sulfur atom and at least one nitrogen atom selected from the group consisting of benzothiazolyl, benzothiadiazolyl, a polycyclic heterocyclic group containing at least one oxygen atom selected from the group consisting of benzofuranyl, isobenzofuranyl, a polycyclic heterocyclic group containing at least one oxygen atom and at least one nitrogen atom selected from the group consisting of benzoxazolyl, benzoxadiazolyl,

$R^2$ and $R^3$ are each $(C_1-C_6)$alkyl, aryl or ar-$(C_1-C_6)$alkyl whereby aryl and the aryl moiety are selected from the group consisting of phenyl, tolyl, xylyl or naphthyl, or $R^2$ and $R^3$ are together to form 1,3-dithian ring,

$R^4$ is hydrogen or $(C_1-C_6)$alkoxy,

and

n is 0 or an integer of 1 to 4, and pharmaceutical acceptable salts thereof,
which comprises reacting a compound of the following formula or its salt :

$R^1-(CH_2)_n-Y$

with a compound of the following formula or its salt :

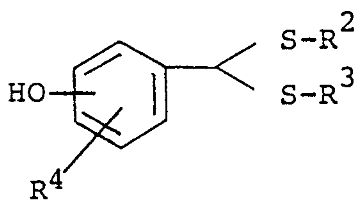

to give a compound of the following formula or its salt :

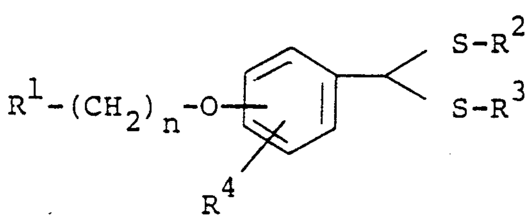

or

reacting a compound of the following formula or its salt :

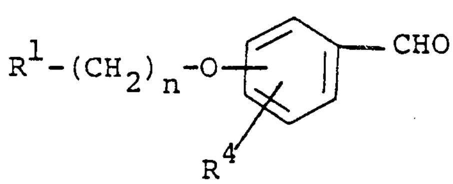

with a compound of the following formula or its salt :

$R^2$-SH

to give a compound of the following formula or its salt :

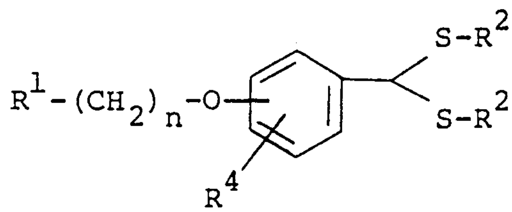

or

reacting a compound of the following formula or its salt :

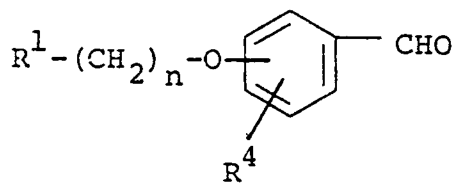

with a compound of the following formula or its salt :

$SH-(CH_2)_3-SH$

to give a compound of the following formula or its salt :

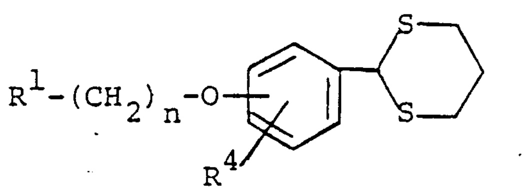

or

reacting a compound of the following formula or its salt :

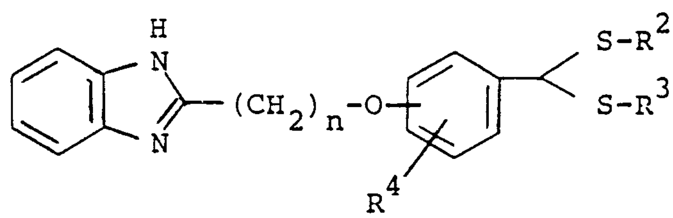

with $(C_1-C_6)$alkyl-alkylating agents to give a compound of the following formula or its salt :

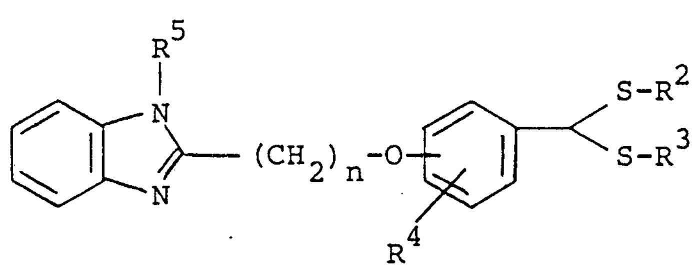

wherein the above formulae $R^1$, $R^2$, $R^3$, $R^4$ and n have the meaning as given in the introductory part of claim 8, $R^5$ is $(C_1-C_6)$alkyl and Y is an acid residue selected from the group consisting of an inorganic

acid residue of an inorganic acid selected from the group consisting of hydrochloric acid, hydrobromic acid, hydroiodic acid, sulfuric acid, of an organic acid selected from the group consisting of sulfonic acid, selected from methanesulfonic acid, benzenesulfonic acid and toluenesulfonic acid, and dimethylcarbamic acid.

**9.** A process according to claim 8,
wherein
$R^1$ is heterocyclic group,
n is 1,
$R^2$ and $R^3$ are each $(C_1\text{-}C_6)$alkyl, and
$R^4$ is hydrogen.

**10.** A process according to claim 9,
wherein the heterocyclic group is unsaturated benzene-fused 5 or 6-membered heterocyclic group containing one or two nitrogen atoms as defined in claim 8.

**11.** A process according to claim 8,
wherein
$R^1$ is quinolyl,
n is 1,
$R^2$ and $R^3$ are each propyl, and
$R^4$ is hydrogen.

**12.** A process according to claim 9,
wherein the heterocyclic group is represented the following formula :

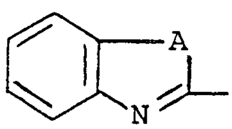

wherein
A is

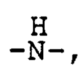

-O- or -S- .

**13.** A process according to claim 8,
wherein $R^1$ is benzothiazolyl, n is 1, $R^2$ and $R^3$ are each propyl, and $R^4$ is hydrogen.

**14.** A pharmaceutical composition comprising as an active ingredient a compound as defined in claim 1 or its pharmaceutically acceptable salt.

**15.** An antiinflammatory agent or antiallergic agent comprising as an active ingredient a compound as defined in claim 1 or its pharmaceutically acceptable salt.

**16.** A compound of claim 1 or pharmaceutical acceptable salts thereof for use as a medicament.

**17.** A compound of claim 1 or pharmaceutical acceptable salts thereof for use in treating or preventing inflammatory or allergic diseases.

**18.** Use of a compound of claim 1 and its pharmaceutically acceptable salt for the manufacture of a medicament for therapeutic treatment of inflammatory or allergic diseases.

**Claims for the following Contracting State : ES**

1. A process for preparation of a dithioacetal compound of the formula:

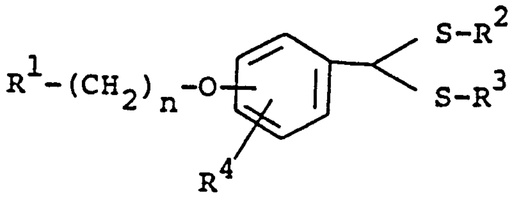

wherein

$R^1$ is $(C_1-C_6)$alkyl, di-$(C_1-C_6)$alkylamino, aryl selected from the group consisting of phenyl, tolyl, xylyl or naphthyl, or heterocyclic group which may have one or more substituents selected from halogen, $(C_1-C_6)$alkyl, $(C_1-C_6)$alkoxy and phenyl, the heterocyclic group being selected from the group consisting of an unsaturated 3- to 8-membered monocyclic heterocyclic group containing 1 to 4 nitrogen atoms selected from the group consisting of pyrrolyl, pyrrolinyl, imidazolyl, pyrazolyl, pyridyl and its N-oxide, dihydropyridyl, pyrimidyl, pyrazinyl, pyridazinyl, triazolyl, a 3- to 8-membered monocyclic heterocyclic group containing at least one sulfur atom and at least one nitrogen atom selected from the group consisting of thiazolyl, isothiazolyl, thiadiazolyl, a polycyclic heterocyclic group containing at least one nitrogen atom selected from the group consisting of indolyl, isoindolyl, indolizinyl, benzimidazolyl, quinolyl, isoquinolyl, quinazolinyl, imidazo(1,2-a)pyridyl, a polycyclic heterocyclic group containing at least one sulfur atom and at least one nitrogen atom selected from the group consisting of benzothiazolyl, benzothiadiazolyl, a polycyclic heterocyclic group containing at least one oxygen atom selected from the group consisting of benzofuranyl, isobenzofuranyl, a polycyclic heterocyclic group containing at least one oxygen atom and at least one nitrogen atom selected from the group consisting of benzoxazolyl, benzoxadiazolyl,

$R^2$ and $R^3$ are each $(C_1-C_6)$alkyl, aryl or ar-$(C_1-C_6)$alkyl whereby aryl and the aryl moiety are selected from the group consisting of phenyl, tolyl, xylyl or naphthyl, or $R^2$ and $R^3$ are together to form 1,3-dithian ring,

$R^4$ is hydrogen or $(C_1-C_6)$alkoxy,

and

n is 0 or an integer of 1 to 4, and pharmaceutical acceptable salts thereof

which comprises reacting a compound of the following formula or its salt :

$R^1-(CH_2)_n-Y$

with a compound of the following formula or its salt :

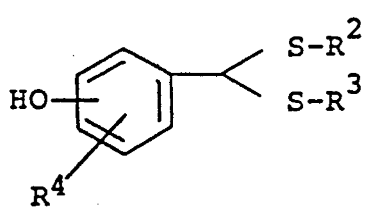

to give a compound of the following formula or its salt :

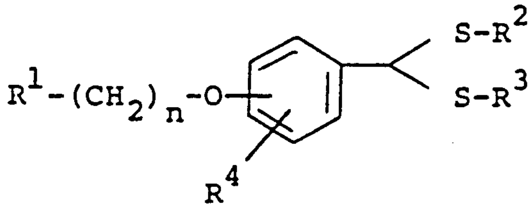

or

reacting a compound of the following formula or its salt :

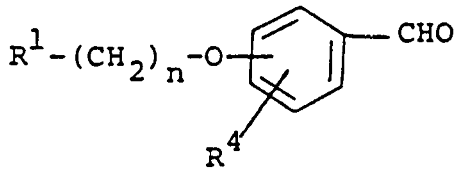

with a compound of the following formula or its salt :

$R^2$-SH

to give a compound of the following formula or its salt :

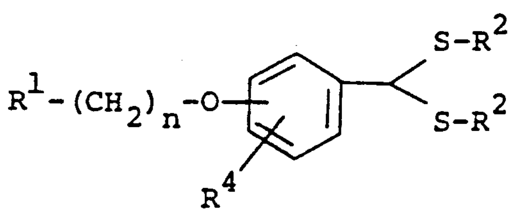

or

reacting a compound of the following formula or its salt :

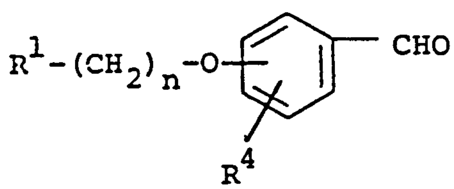

with a compound of the following formula or its salt :

SH-$(CH_2)_3$-SH

to give a compound of the following formula or its salt :

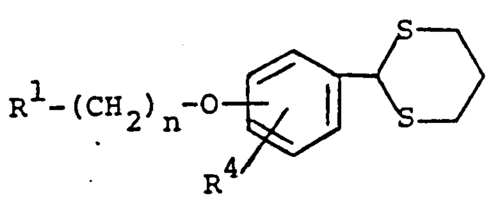

or

reacting a compound of the following formula or its salt :

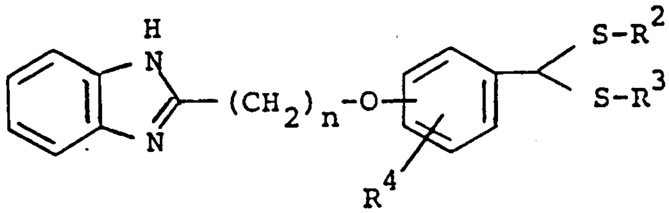

with ($C_1$-$C_6$)alkyl-alkylating agents to give a compound of the following formula or its salt :

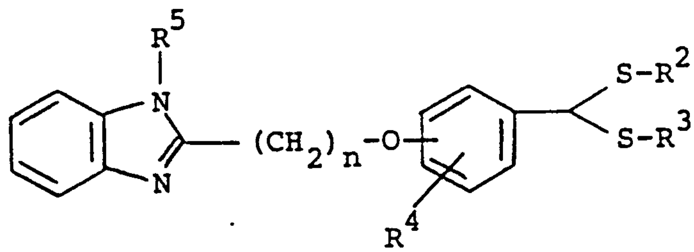

wherein in the above formulae $R^1$, $R^2$, $R^3$, $R^4$ and n have the meaning given above, $R^5$ is ($C_1$-$C_6$)alkyl and Y is an acid residue selected from the group consisting of an inorganic acid residue of an inorganic acid selected from the group consisting of hydrochloric acid, hydrobromic acid, hydroiodic acid, sulfuric acid, of an organic acid selected from the group consisting of sulfonic acid, selected from methanesulfonic acid, benzenesulfonic acid and toluenesulfonic acid, and dimethylcarbamic acid.

**2.** The process according to claim 1,
wherein
$R^1$ is a heterocyclic group as defined in claim 1
n is 1,
$R^2$ and $R^3$ are each ($C_1$-$C_6$)alkyl, and
$R^4$ is hydrogen.

**3.** The process according to claim 1, wherein the compound is represented by the following formula:

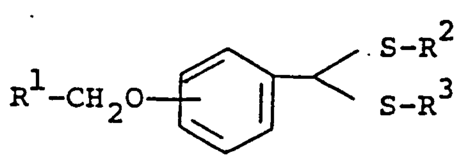

wherein $R^1$ is a heterocyclic group as defined in claim 1 which may have one or more substituents selected from halogen, $C_1$-$C_6$)alkyl, ($C_1$-$C_6$)alkoxy and phenyl, and
$R^2$ and $R^3$ are each ($C_1$-$C_6$)alkyl.

**4.** The process according to claim 1,
wherein the compound is represented by the following formula :

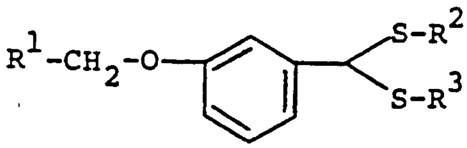

wherein

$R^1$ is unsaturated benzene-fused 5 or 6-membered heterocyclic group containing one or two nitrogen atoms as defined in claim 1, and
$R^2$ and $R^3$ are each $(C_1-C_6)$alkyl.

**5.** The process according to claim 4,
wherein
$R^1$ is quinolyl, and
$R^2$ and $R^3$ are each propyl.

**6.** The process according to claim 4,
wherein $R^1$ is a heterocyclic group of the formula :

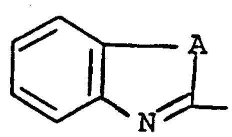

wherein
A is

$$-\overset{H}{N}-,$$

-O- or -S- and
$R^2$ and $R^3$ are each $(C_1-C_6)$alkyl.

**7.** The process according to claim 6,
wherein
$R^1$ is benzothiazolyl, and
$R^2$ and $R^3$ are each propyl.

**8.** The process according to claim 2,
wherein the heterocyclic group is unsaturated benzene-fused 5 or 6-membered heterocyclic group containing one or two nitrogen atoms as defined in claim 1.

**9.** The process according to claim 1,
wherein
$R^1$ is quinolyl,
n is 1,
$R^2$ and $R^3$ are each propyl, and
$R^4$ is hydrogen.

**10.** The process according to claim 2,
wherein the heterocyclic group is represented the following formula :

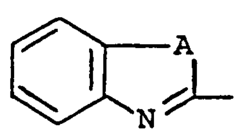

wherein
A is

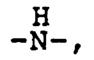

-O- or -S- .

**11.** The process according to claim 1, wherein $R^1$ is benzothiazolyl, n is 1, $R^2$ and $R^3$ are each propyl, and $R^4$ is hydrogen.

**Claims for the following Contracting State : GR**

**1.** A process for preparation of a dithioacetal compound of the formula:

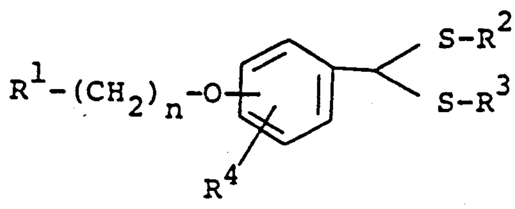

(I)

wherein

$R^1$ is $(C_1\text{-}C_6)$alkyl, di-$(C_1\text{-}C_6)$alkylamino, aryl selected from the group consisting of phenyl, tolyl, xylyl or naphthyl, or heterocyclic group which may have one or more substituents selected from halogen, $(C_1\text{-}C_6)$alkyl, $(C_1\text{-}C_6)$alkoxy and phenyl, the heterocyclic group being selected from the group consisting of an unsaturated 3- to 8-membered monocyclic heterocyclic group containing 1 to 4 nitrogen atoms selected from the group consisting of pyrrolyl, pyrrolinyl, imidazolyl, pyrazolyl, pyridyl and its N-oxide, dihydropyridyl, pyrimidyl, pyrazinyl, pyridazinyl, triazolyl, a 3- to 8-membered monocyclic heterocyclic group containing at least one sulfur atom and at least one nitrogen atom selected from the group consisting of thiazolyl, isothiazolyl, thiadiazolyl, a polycyclic heterocyclic group containing at least one nitrogen atom selected from the group consisting of indolyl, isoindolyl, indolizinyl, benzimidazolyl, quinolyl, isoquinolyl, quinazolinyl, imidazo(1,2-a)pyridyl, a polycyclic heterocyclic group containing at least one sulfur atom and at least one nitrogen atom selected from the group consisting of benzothiazolyl, benzothiadiazolyl, a polycyclic heterocyclic group containing at least one oxygen atom selected from the group consisting of benzofuranyl, isobenzofuranyl, a polycyclic heterocyclic group containing at least one oxygen atom and at least one nitrogen atom selected from the group consisting of benzoxazolyl, benzoxadiazolyl,

$R^2$ and $R^3$ are each $(C_1\text{-}C_6)$alkyl, aryl or ar-$(C_1\text{-}C_6)$alkyl whereby aryl and the aryl moiety are selected from the group consisting of phenyl, tolyl, xylyl or naphthyl, or $R^2$ and $R^3$ are together to form 1,3-dithian ring,

$R^4$ is hydrogen or $(C_1\text{-}C_6)$alkoxy,

and

n is 0 or an integer of 1 to 4, and pharmaceutical acceptable salts thereof

which comprises reacting a compound of the following formula or its salt :

$R^1\text{-}(CH_2)_n\text{-}Y$

with a compound of the following formula or its salt :

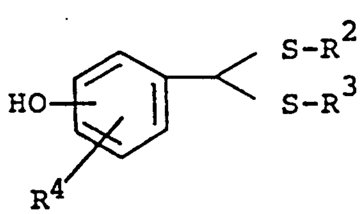

to give a compound of the following formula or its salt :

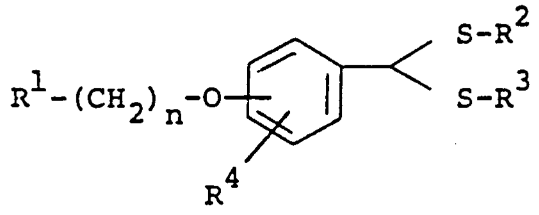 or

reacting a compound of the following formula or its salt :

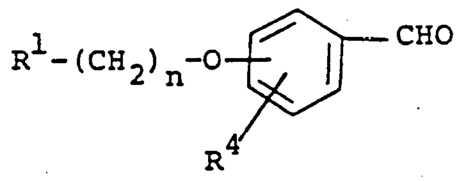

with a compound of the following formula or its salt :

$R^2$-SH

to give a compound of the following formula or its salt :

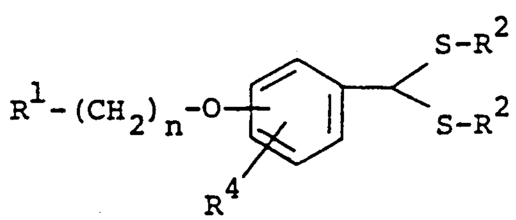 or

reacting a compound of the following formula or its salt :

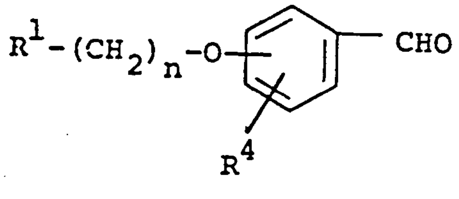

with a compound of the following formula or its salt :

$SH-(CH_2)_3-SH$

to give a compound of the following formula or its salt :

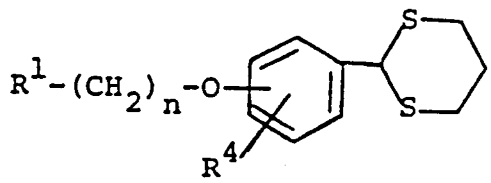

or

reacting a compound of the following formula or its salt :

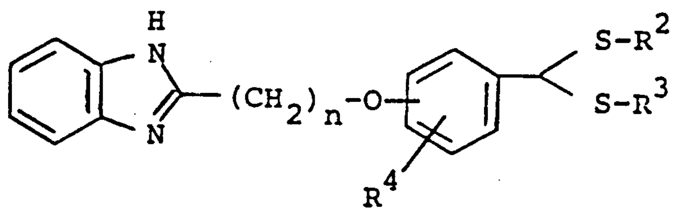

with ($C_1$-$C_6$)alkyl-alkylating agents to give a compound of the following formula or its salt :

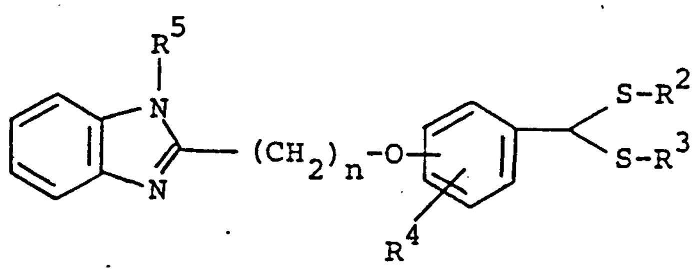

wherein in the above formulae $R^1$, $R^2$, $R^3$, $R^4$ and n have the meaning given above, $R^5$ is ($C_1$-$C_6$)alkyl and Y is an acid residue selected from the group consisting of an inorganic acid residue of an inorganic acid selected from the group consisting of hydrochloric acid, hydrobromic acid, hydroiodic acid, sulfuric acid, of an organic acid selected from the group consisting of sulfonic acid, selected from methanesulfonic acid, benzenesulfonic acid and toluenesulfonic acid, and dimethylcarbamic acid.

**2.** The process according to claim 1,
wherein
$R^1$ is a heterocyclic group as defined in claim 1
n is 1,
$R^2$ and $R^3$ are each ($C_1$-$C_6$)alkyl, and
$R^4$ is hydrogen.

**3.** The process according to claim 1, wherein the compound is represented by the following formula:

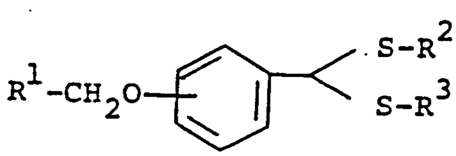

wherein $R^1$ is a heterocyclic group as defined in claim 1 which may have one or more substituents selected from halogen, $C_1$-$C_6$)alkyl, ($C_1$-$C_6$)alkoxy and phenyl, and
$R^2$ and $R^3$ are each ($C_1$-$C_6$)alkyl.

**4.** The process according to claim 1,

wherein the compound is represented by the following formula :

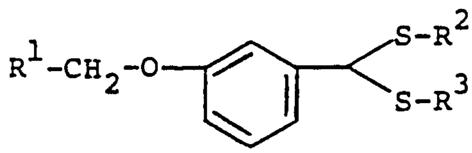

wherein
$R^1$ is unsaturated benzene-fused 5 or 6-membered heterocyclic group containing one or two nitrogen atoms as defined in claim 1, and
$R^2$ and $R^3$ are each $(C_1-C_6)$alkyl.

**5.** The process according to claim 4,
wherein
$R^1$ is quinolyl, and
$R^2$ and $R^3$ are each propyl.

**6.** The process according to claim 4,
wherein $R^1$ is a heterocyclic group of the formula :

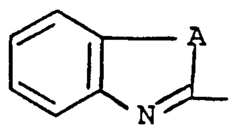

wherein
A is

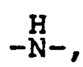

-O- or S, and
$R^2$ and $R^3$ are each $(C_1-C_6)$alkyl.

**7.** The process according to claim 6,
wherein
$R^1$ is benzothiazolyl, and $R^2$ and $R^3$ are each propyl.

**8.** The process according to claim 2,
wherein the heterocyclic group is unsaturated benzene-fused 5 or 6-membered heterocyclic group containing one or two nitrogen atoms as defined in claim 1.

**9.** The process according to claim 1,
wherein
$R^1$ is quinolyl,
n is 1,
$R^2$ and $R^3$ are each propyl, and
$R^4$ is hydrogen.

**10.** The process according to claim 2,
wherein the heterocyclic group is represented the following formula :

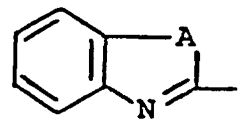

wherein
A is

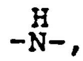

-O- or -S- .

**11.** The process according to claim 1, wherein $R^1$ is benzothiazolyl, n is 1, $R^2$ and $R^3$ are each propyl, and $R^4$ is hydrogen.

**12.** Modification of the process claimed in claim 1 which is characterized by bringing a compound of formula (I), or a non-toxic salt thereof, produced by a process claimed in claim 1, into pharmaceutically acceptable form by admixture or presentation of said compound with a pharmaceutically acceptable diluent or carrier.

**Revendications**
**Revendications pour les Etats contractants suivants : AT, BE, CH, DE, FR, GB, IT, LI, LU, NL, SE**

**1.** Composé de dithioacétal de la formule

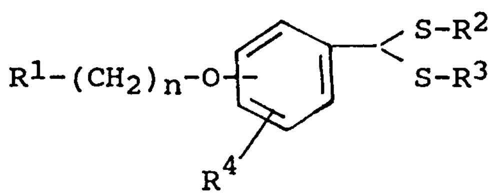

où

$R^1$ est un $(C_1-C_6)$alkyle, un di-$(C_1-C_6)$alkylamino, un aryle choisi dans le groupe constitué du phényle, du tolyle, du xylyle, ou du naphtyle, ou un groupe hétérocyclique qui peut avoir un ou plusieurs substituants choisis parmi l'halogène, le $(C_1-C_6)$alkyle, le $(C_1-C_6)$alkoxy et le phényle, le groupe hétérocyclique étant choisi dans le groupe des hétérocycliques monocycliques insaturés à 3 - 8 éléments contenant 1 à 4 atomes d'azote, constitué du pyrrolyle, du pyrrolinyle, de l'imidazolyle, du pyrazolyle, du pyridyle et de son N-oxyde, du dihydropyridyle, du pyrimidyle, du pyrazinyle, du pyridazinyle, du triazolyle, le groupe des hétérocycliques monocycliques à 3 - 8 éléments contenant au moins un atome de soufre et au moins un atome d'azote, constitué du thiazolyle, de l'isothiazolyle, du thiadiazolyle, le groupe des hétérocycliques polycycliques contenant au moins un atome d'azote, constitué de l'indolyle, de l'isoindolyle, de l'indolizinyle, du benzimidazolyle, du quinolyle, de l'isoquinolyle, du quinazolinyle, de l'imidazo(1,2-a)pyridyle, le groupe des hétérocycliques polycycliques contenant au moins un atome de soufre et au moins un atome d'azote, constitué du benzothiazolyle, du benzothiadiazolyle, le groupe des hétérocycliques polycycliques contenant au moins un atome d'oxygène, constitué du benzofuranyle, de l'isobenzofuranyle, le groupe des hétérocycliques polycycliques contenant au moins un atome d'oxygène et au moins un atome d'azote, constitué du benzoxazolyle, du benzoxadiazolyle,

$R^2$ et $R^3$ sont chacun un $(C_1-C_6)$alkyle, un aryle ou un ar-$(C_1-C_6)$alkyle, où l'aryle et le groupement aryle sont choisis dans un groupe comprenant le phényle, le tolyle, le xylyle ou le naphtyle, ou $R^2$ et $R^3$ sont réunis pour former un cycle 1,3-dithia

$R^4$ est un hydrogène ou un $(C_1-C_6)$alkoxy,

et

n est égal à 0 ou à un nombre entier de 1 à 4, et ses sels pharmaceutiquement acceptables.

**2.** Composé selon la revendication 1, dans lequel
$R^1$ est un groupe hétérocyclique comme défini dans la revendication 1,
n est égal à 1,
$R^2$ et $R^3$ sont chacun un $(C_1-C_6)$alkyle, et
$R^4$ est un hydrogène.

**3.** Composé selon la revendication 1, dans lequel le composé est représenté par la formule suivante :

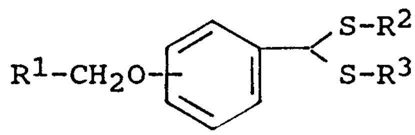

où
$R^1$ est un groupe hétérocyclique comme défini dans la revendication 1, qui peut avoir un ou plusieurs substituants choisis parmi l'halogène, le $(C_1-C_6)$alkyle, le $(C_1-C_6)$alkoxy et le phényle, et
$R^2$ et $R^3$ sont chacun un $(C_1-C_6)$alkyle.

**4.** Composé selon la revendication 1,
dans lequel le composé est représenté par la formule suivante :

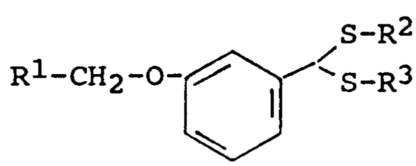

où
$R^1$ est un groupe hétérocyclique insaturé aromatique à 5 ou 6 éléments, contenant un ou deux atomes d'azote comme défini dans la revendication 1, et
$R^2$ et $R^3$ sont chacun un $(C_1-C_6)$alkyle.

**5.** Composé selon la revendication 4, dans lequel
$R^1$ est un quinolyle, et
$R^2$ et $R^3$ sont chacun un propyle.

**6.** Composé selon la revendication 4, dans lequel
$R^1$ est un groupe hétérocyclique de la formule :

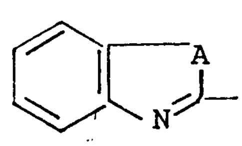

où
A est

H
-N-,

-O- ou -S-, et
$R^2$ et $R^3$ sont chacun un $(C_1-C_6)$alkyle.

7. Composé selon la revendication 6, dans lequel
$R^1$ est un benzothiazolyle, et
$R^2$ et $R^3$ sont chacun un propyle.

8. Procédé de préparation d'un composé de la formule suivante :

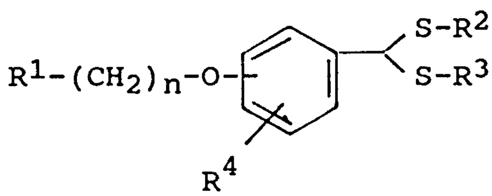

où
$R^1$ est un $(C_1\text{-}C_6)$alkyle, un di-$(C_1\text{-}C_6)$alkylamino, un aryle choisi dans le groupe constitué du phényle, du tolyle, du xylyle, ou du naphtyle, ou un groupe hétérocyclique qui peut avoir un ou plusieurs substituants choisis parmi l'halogène, le $(C_1\text{-}C_6)$alkyle, le $(C_1\text{-}C_6)$alkoxy et le phényle, le groupe hétérocyclique étant choisi dans le groupe des hétérocycliques monocycliques insaturés à 3 - 8 éléments contenant 1 à 4 atomes d'azote, constitué du pyrrolyle, du pyrrolinyle, de l'imidazolyle, du pyrazolyle, du pyridyle et de son N-oxyde, du dihydropyridyle, du pyrimidyle, du pyrazinyle, du pyridazinyle, du triazolyle, le groupe des hétérocycliques monocycliques à 3 - 8 éléments contenant au moins un atome de soufre et au moins un atome d'azote, constitué du thiazolyle, de l'isothiazolyle, du thiadiazolyle, le groupe des hétérocycliques polycycliques contenant au moins un atome d'azote, constitué de l'indolyle, de l'isoindolyle, de l'indolizinyle, du benzimidazolyle, du quinolyle, de l'isoquinolyle, du quinazolinyle, de l'imidazo(1,2-a)pyridyle, le groupe des hétérocycliques polycycliques contenant au moins un atome de soufre et au moins un atome d'azote, constitué du benzothiazolyle, du benzothiadiazolyle, le groupe des hétérocycliques polycycliques contenant au moins un atome d'oxygène, constitué du benzofuranyle, de l'isobensofuranyle, le groupe des hétérocycliques polycycliques contenant au moins un atome d'oxygène et au moins un atome d'azote, constitué du benzoxazolyle, du benzoxadiazolyle,
$R^2$ et $R^3$ sont chacun un $(C_1\text{-}C_6)$alkyle, un aryle ou un ar-$(C_1\text{-}C_6)$alkyle, où l'aryle et le groupement aryle sont choisis dans un groupe comprenant le phényle, le tolyle, le xylyle ou le naphtyle ou $R^2$ et $R^3$ sont réunis pour former un cycle 1,3-dithia
$R^4$ est un hydrogène ou un $(C_1\text{-}C_6)$alkoxy,
et
n est égal à 0 ou à un nombre entier de 1 à 4, et ses sels pharmaceutiquement acceptables,
qui comprend de faire réagir un composé de la formule suivante ou son sel :

$R^1\text{-}(CH_2)_n\text{-}Y$

avec un composé de la formule suivante ou son sel :

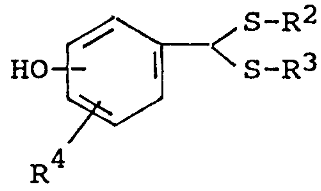

pour fournir un composé de la formule suivante ou son sel :

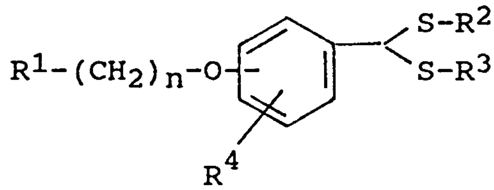

ou

de faire réagir un composé de la formule suivante ou son sel :

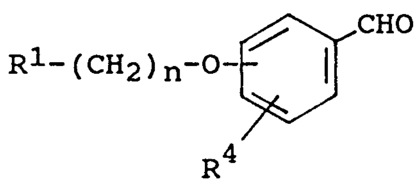

avec un composé de la formule suivante ou son sel :

$R^2$-SH

pour fournir un composé de la formule suivante ou son sel :

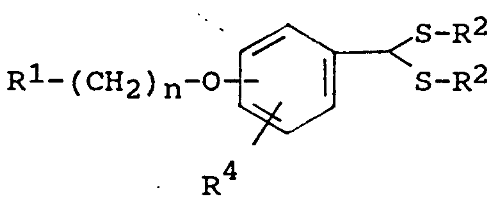

ou

de faire réagir un Composé de la formule suivante ou son sel :

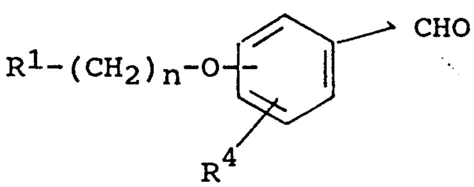

avec un composé de la formule suivante ou son sel :

$SH-(CH_2)_3-SH$

pour fournir un composé de la formule suivante ou son sel :

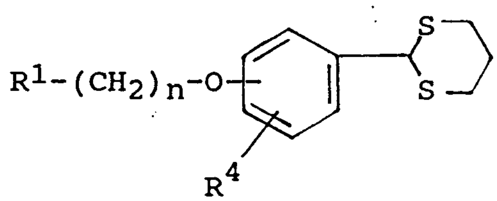

ou

de faire réagir un composé de la formule suivante ou son sel :

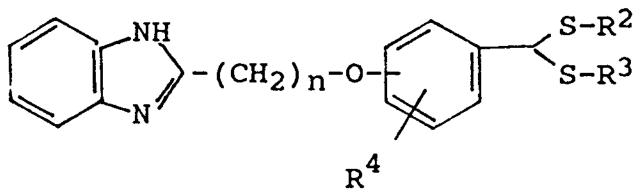

avec des agents d'alkylation ($C_1$-$C_6$)alkyle pour fournir un composé de la formule suivante ou son sel :

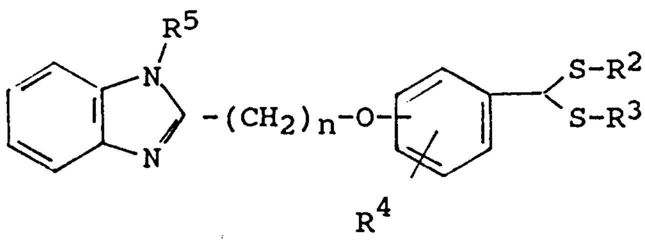

où dans les formules ci-dessus, $R^1$, $R^2$, $R^3$ , $R^4$ et n ont la signification donnée dans la partie d'introduction de la revendication 8, $R^5$ est un ($C_1$-$C_6$)alkyle et Y est une fonction acide choisie dans le groupe des fonctions acides dérivées des acides minéraux du groupe constitué par l'acide chlorhydrique, l'acide bromhydrique, l'acide iodhydrique, l'acide sulfurique, des acides organiques sulfoniques du groupe constitué par l'acide méthanesulfonique, benzènesulfonique et toluènesulfonique, et de l'acide diméthylcarbamique.

**9.** Procédé selon la revendication 8, dans lequel
$R^1$ est un groupe hétérocyclique,
n est égal à 1,
$R^2$ et $R^3$ sont chacun un ($C_1$-$C_6$)alkyle, et
$R^4$ est un hydrogène.

**10.** Procédé selon la revendication 9, dans lequel
le groupe hétérocyclique est un groupe hétérocyclique insaturé aromatique à 5 ou 6 éléments, contenant un ou deux atomes d'azote comme défini dans la revendication 8.

**11.** Procédé selon la revendication 8, dans lequel
$R^1$ est un quinolyle,
n est égal à 1,
$R^2$ et $R^3$ sont chacun un propyle, et
$R^4$ est un hydrogène.

**12.** Procédé selon la revendication 9, dans lequel
le groupe hétérocyclique est représenté par la formule suivante :

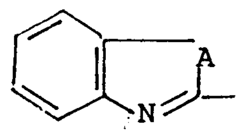

où
A est

$$-\overset{H}{N}-,$$

-O- ou -S-.

**13.** Procédé selon la revendication 8, dans lequel
$R^1$ est un benzothiazolyle, n est égal à 1, $R^2$ et $R^3$ sont chacun un propyle et $R^4$ est un hydrogène.

**14.** Composition pharmaceutique comprenant comme ingrédient actif un composé comme défini dans la revendication 1, ou son sel pharmaceutiquement acceptable.

**15.** Agent anti-inflammatoire ou agent anti-allergique comprenant comme ingrédient actif un composé comme défini dans la revendication 1 ou son sel pharmaceutiquement acceptable.

**16.** Composé selon la revendication 1 ou ses sels pharmaceutiquement acceptables pour une utilisation en tant que médicament.

**17.** Composé selon la revendication 1 ou ses sels pharmaceutiquement acceptables pour une utilisation dans le traitement ou la prévention de maladies inflammatoires ou allergiques.

**18.** Utilisation d'un composé selon la revendication 1 et son sel pharmaceutiquement acceptable pour la fabrication d'un médicament pour le traitement thérapeutique de maladies inflammatoire ou allergiques

**Revendications pour l'Etat contractant suivant : ES**

**1.** Procédé de préparation d'un composé de dithioacétal de la formule :

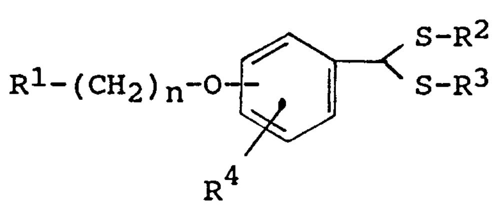

où
$R^1$ est un $(C_1\text{-}C_6)$alkyle, un di-$(C_1\text{-}C_6)$alkylamino, un aryle choisi dans le groupe constitué du phényle, du tolyle, du xylyle, ou du naphtyle, ou un groupe hétérocyclique qui peut avoir un ou plusieurs substituants choisis parmi l'halogène, le $(C_1\text{-}C_6)$alkyle, le $(C_1\text{-}C_6)$alkoxy et le phényle, le groupe hétérocyclique étant choisi dans le groupe des hétérocycliques monocycliques insaturés à 3 - 8 éléments contenant 1 à 4 atomes d'azote, constitué du pyrrolyle, du pyrrolinyle, de l'imidazolyle, du pyrazolyle, du pyridyle et de son N-oxyde, du dihydropyridyle, du pyrimidyle, du pyrazinyle, du pyridazinyle, du triazolyle, le groupe des hétérocycliques monocycliques à 3 - 8 éléments contenant au moins un atome de soufre et au moins un atome d'azote, constitué du thiazolyle, de l'isothiazolyle, du thiadiazolyle, le groupe des hétérocycliques polycycliques contenant au moins un atome d'azote, constitué de l'indolyle, de l'isoindolyle, de l'indolizinyle, du benzimidazolyle, du quinolyle, de l'isoquinolyle, du quinazolinyle, de l'imidazo(1,2-a)pyridyle, le groupe des hétérocycliques polycycliques contenant au moins un atome de soufre et au moins un atome d'azote, constitué du benzothiazolyle, du benzothiadiazolyle, le groupe des hétérocycliques polycycliques contenant au moins un atome d'oxygène, constitué du benzofuranyle, de l'isobenzofuranyle, le groupe des hétérocycliques polycycliques contenant au moins un atome d'oxygène et au moins un atome d'azote, constitué du benzoxazolyle, du benzoxadiazolyle,

$R^2$ et $R^3$ sont chacun un ($C_1$-$C_6$)alkyle, un aryle ou un ar-($C_1$-$C_6$)alkyle, où l'aryle et le groupement aryle sont choisis dans un groupe comprenant le phényle, le tolyle, le xylyle ou le naphtyle, ou $R^2$ et $R^3$ sont réunis pour former un cycle 1,3-dithia

$R^4$ est un hydrogène ou un ($C_1$-$C_6$)alkoxy,

et

n est égal à 0 ou à un nombre entier de 1 à 4, et ses sels pharmaceutiquement acceptables,
qui comprend de faire réagir un composé de la formule suivante ou son sel :

$R^1$-$(CH_2)_n$-Y

avec un composé de la formule suivante ou son sel :

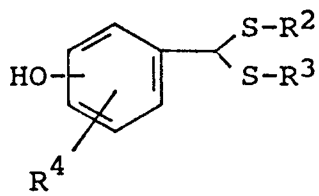

pour fournir un composé de la formule suivante ou son sel :

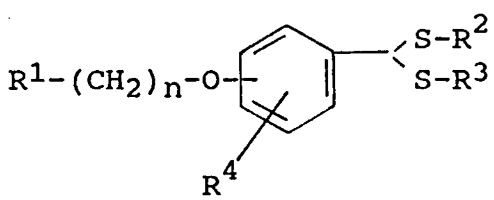

ou

de faire réagir un composé de la formule suivante ou son sel :

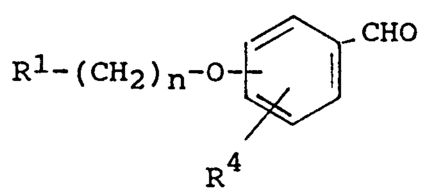

avec un composé de la formule suivante ou son sel :

$R^2$-SH

pour fournir un composé de la formule suivante ou son sel :

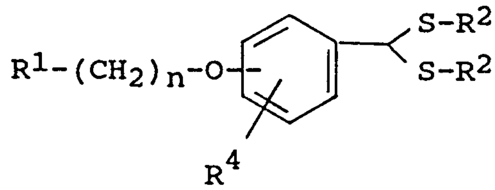

ou

de faire réagir un composé de la formule suivante ou son sel :

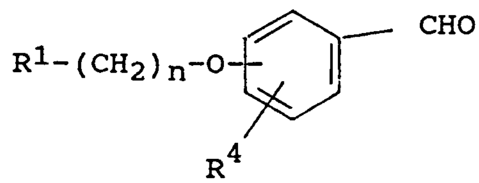

avec un composé de la formule suivante ou son sel :

$SH-(CH_2)_3-SH$

pour fournir un composé de la formule suivante ou son sel :

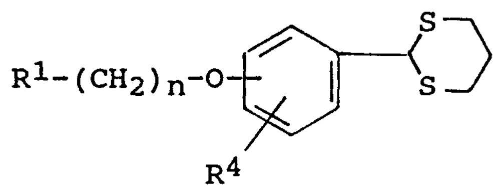

ou

de faire réagir un composé de la formule suivante ou son sel :

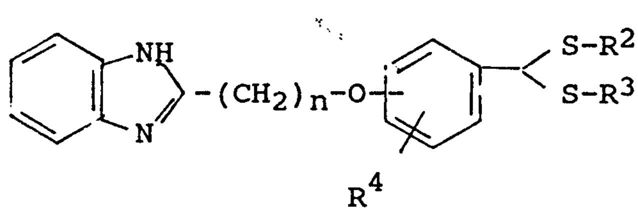

avec des agents d'alkylation ($C_1$-$C_6$)alkyle pour fournir un composé de la formule suivante ou son sel :

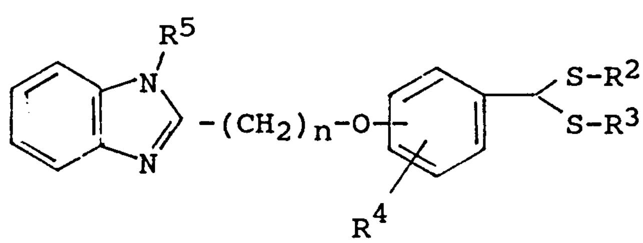

où dans les formules ci-dessus, $R^1$, $R^2$, $R^3$ , $R^4$ et n ont la signification donnée ci-dessus, $R^5$ est un ($C_1$-$C_6$)alkyle et Y est une fonction acide choisie dans le groupe des fonctions acides dérivées des acides minéraux du groupe constitué par l'acide chlorhydrique, l'acide bromhydrique, l'acide iodhydrique, l'acide sulfurique, des acides organiques sulfoniques du groupe constitué par l'acide méthanesulfonique, benzènesulfonique et toluènesulfonique, et de l'acide diméthylcarbamique.

**2.** Procédé selon la revendication 1, dans lequel
$R^1$ est un groupe hétérocyclique, comme défini dans la revendication 1,
n est égal à 1,
$R^2$ et $R^3$ sont chacun un ($C_1$-$C_6$)alkyle, et
$R^4$ est un hydrogène.

**3.** Procédé selon la revendication 1, dans lequel le composé est représenté par la formule suivante :

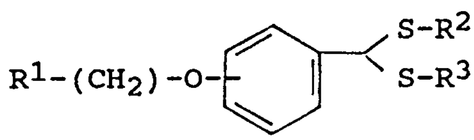

où $R^1$ est un groupe hétérocyclique comme défini dans la revendication 1, qui peut avoir un ou plusieurs substituants choisis parmi l'halogène, le ($C_1$-$C_6$)alkyle, le ($C_1$-$C_6$)alkoxy et le phényle, et
$R^2$ et $R^3$ sont chacun un ($C_1$-$C_6$)alkyle.

**4.** Procédé selon la revendication 1, dans lequel le composé est représenté par la formule suivante :

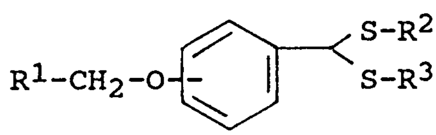

où
$R^1$ est un groupe hétérocyclique insaturé aromatique à 5 ou 6 éléments contenant un ou deux atomes d'azote, comme défini dans la revendication 1, et
$R^2$ et $R^3$ sont chacun un ($C_1$-$C_6$)alkyle.

**5.** Procédé selon la revendication 4, dans lequel
$R^1$ est un quinolyle, et
$R^2$ et $R^3$ sont chacun un propyle.

**6.** Procédé selon la revendication 4, dans lequel
$R^1$ est un groupe hétérocyclique de la formule :

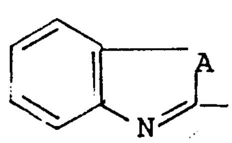

où
A est

H
-N-,

-O- ou -S-, et
$R^2$ et $R^3$ sont chacun un ($C_1$-$C_6$)alkyle.

**7.** Procédé selon la revendication 6, dans lequel
$R^1$ est un benzothiazolyle, et
$R^2$ et $R^3$ sont chacun un propyle.

**8.** Procédé selon la revendication 2, dans lequel le groupe hétérocyclique est un groupe hétérocyclique insaturé aromatique à 5 ou 6 éléments contenant un ou deux atomes d'azote, comme défini dans la revendication 1.

**9.** Procédé selon la revendication 1, dans lequel
$R^1$ est un quinolyle,
n est égal à 1,
$R^2$ et $R^3$ sont chacun un propyle, et
$R^4$ est un hydrogène.

**10.** Procédé selon la revendication 2, dans lequel le groupe hétérocyclique est représenté par la formule suivante :

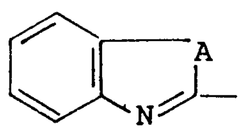

où
A est

H
-N-,

-O- ou -S-.

**11.** Procédé selon la revendication 1, dans lequel $R^1$ est le benzothiazolyle, n est égal à 1, $R^2$ et $R^3$ sont chacun un propyle et $R^4$ est un hydrogène.

**Revendications pour l'Etat contractant suivant : GR**

**1.** Procédé de préparation d'un composé de dithioacétal de la formule :

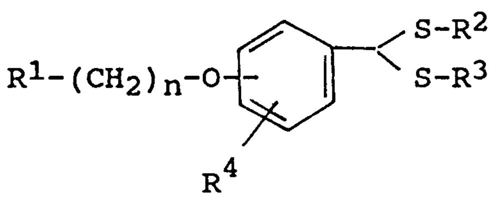

(I)

où
$R^1$ est un ($C_1$-$C_6$)alkyle, un di-($C_1$-$C_6$)alkylamino, un aryle choisi dans le groupe constitué du

phényle, du tolyle, du xylyle, ou du naphtyle, ou un groupe hétérocyclique qui peut avoir un ou plusieurs substituants choisis parmi l'halogène, le ($C_1$-$C_6$)alkyle, le ($C_1$-$C_6$)alkoxy et le phényle, le groupe hétérocyclique étant choisi dans le groupe des hétérocycliques monocycliques insaturés à 3 - 8 éléments contenant 1 à 4 atomes d'azote, constitué du pyrrolyle, du pyrrolinyle, de l'imidazolyle, du pyrazolyle, du pyridyle et de son N-oxyde, du dihydropyridyle, du pyrimidyle, du pyrazinyle, du pyridazinyle, du triazolyle, le groupe des hétérocycliques monocycliques à 3 - 8 éléments contenant au moins un atome de soufre et au moins un atome d'azote, constitué du thiazolyle, de l'isothiazolyle, du thiadiazolyle, le groupe des hétérocycliques polycycliques contenant au moins un atome d'azote, constitué de l'indolyle, de l'isoindolyle, de l'indolizinyle, du benzimidazolyle, du quinolyle, de l'isoquinolyle, du quinazolinyle, de l'imidazo(1,2-a)pyridyle, le groupe des hétérocycliques polycycliques contenant au moins un atome de soufre et au moins un atome d'azote, constitué du benzothiazolyle, du benzothiadiazolyle, le groupe des hétérocycliques polycycliques contenant au moins un atome d'oxygène, constitué du benzofuranyle, de l'isobenzofuranyle, le groupe des hétérocycliques polycycliques contenant au moins un atome d'oxygène et au moins un atome d'azote, constitué du benzoxazolyle, du benzoxadiazolyle,

$R^2$ et $R^3$ sont chacun un ($C_1$-$C_6$)alkyle, un aryle ou un ar-($C_1$-$C_6$)alkyle, où l'aryle et le groupement aryle sont choisis dans un groupe comprenant le phényle, le tolyle, le xylyle ou le naphtyle, ou $R^2$ et $R^3$ sont réunis pour former un cycle 1,3-dithia

$R^4$ est un hydrogène ou un ($C_1$-$C_6$)alkoxy,

et

n est égal à 0 ou à un nombre entier de 1 à 4, et ses sels pharmaceutiquement acceptables,
qui comprend de faire réagir un composé de la formule suivante ou son sel :

$R^1$-$(CH_2)_n$-Y

avec un composé de la formule suivante ou son sel :

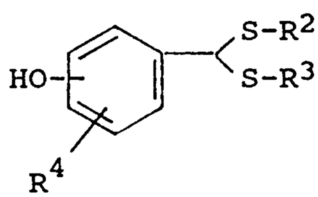

pour fournir un composé de la formule suivante ou son sel :

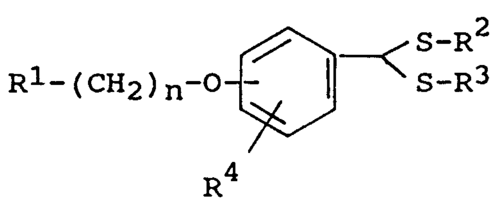

ou

de faire réagir un composé de la formule suivante ou son sel :

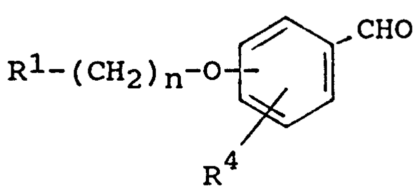

avec un composé de la formule suivante ou son sel :

$R^2$-SH

pour fournir un composé de la formule suivante ou son sel :

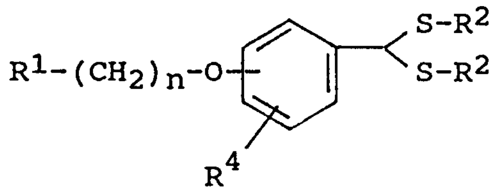

ou

de faire réagir un composé de la formule suivante ou son sel :

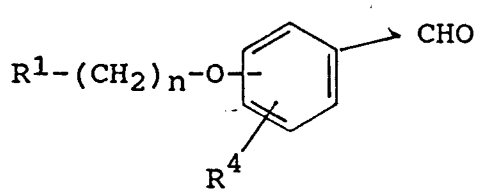

avec un composé de la formule suivante ou son sel :

$SH-(CH_2)_3-SH$

pour fournir un composé de la formule suivante ou son sel :

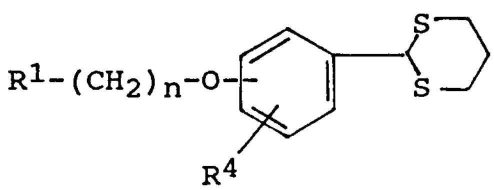

ou

de faire réagir un composé de la formule suivante ou son sel :

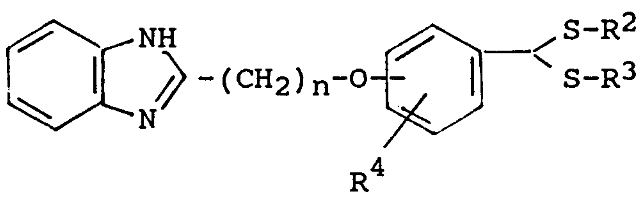

avec des agents d'alkylation ($C_1$-$C_6$)alkyle pour fournir un composé de la formule suivante ou son sel :

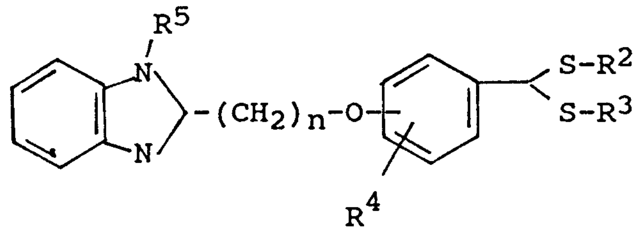

où dans les formules ci-dessus, $R^1$, $R^2$, $R^3$ , $R^4$ et n ont la signification donnée ci-dessus, $R^5$ est un ($C_1$-$C_6$)alkyle et Y est une fonction acide choisie dans le groupe des fonctions acides dérivées des acides minéraux du groupe constitué par l'acide chlorhydrique, l'acide bromhydrique, l'acide iodhydrique, l'acide sulfurique, des acides organiques sulfoniques du groupe constitué par l'acide méthanesulfonique, benzènesulfonique et toluènesulfonique, et de l'acide diméthylcarbamique.

**2.** Procédé selon la revendication 1, dans lequel
$R^1$ est un groupe hétérocyclique, comme défini dans la revendication 1,
n est égal à 1,
$R^2$ et $R^3$ sont chacun un ($C_1$-$C_6$)alkyle, et
$R^4$ est un hydrogène.

**3.** Procédé selon la revendication 1, dans lequel le composé est représenté par la formule suivante :

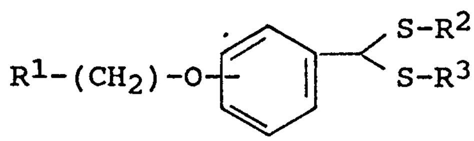

où $R^1$ est un groupe hétérocyclique comme défini dans la revendication 1, qui peut avoir un ou plusieurs substituants choisis parmi l'halogène, le ($C_1$-$C_6$)alkyle, le ($C_1$-$C_6$)alkoxy et le phényle, et
$R^2$ et $R^3$ sont chacun un ($C_1$-$C_6$)alkyle.

**4.** Procédé selon la revendication 1, dans lequel le composé est représenté par la formule suivante :

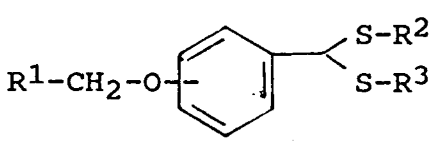

où
$R^1$ est un groupe hétérocyclique insaturé aromatique à 5 ou 6 éléments contenant un ou deux atomes d'azote, comme défini dans la revendication 1, et
$R^2$ et $R^3$ sont chacun un ($C_1$-$C_6$)alkyle.

**5.** Procédé selon la revendication 4, dans lequel
$R^1$ est un quinolyle, et
$R^2$ et $R^3$ sont chacun un propyle.

**6.** Procédé selon la revendication 4, dans lequel
$R^1$ est un groupe hétérocyclique de la formule :

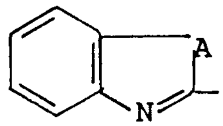

où
A est

$$-\overset{H}{N}-,$$

-O- ou -S-, et
$R^2$ et $R^3$ sont chacun un $(C_1\text{-}C_6)$alkyle.

**7.** Procédé selon la revendication 6, dans lequel
$R^1$ est un benzothiazolyle, et
$R^2$ et $R^3$ sont chacun un propyle.

**8.** Procédé selon la revendication 2, dans lequel le groupe hétérocyclique est un groupe hétérocyclique insaturé aromatique à 5 ou 6 éléments contenant un ou deux atomes d'azote, comme défini dans la revendication 1.

**9.** Procédé selon la revendication 1, dans lequel
$R^1$ est un quinolyle,
n est égal à 1,
$R^2$ et $R^3$ sont chacun un propyle, et
$R^4$ est un hydrogène.

**10.** Procédé selon la revendication 2, dans lequel le groupe hétérocyclique est représenté par la formule suivante :

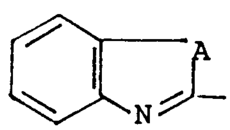

où
A est

$$-\overset{H}{N}-,$$

-O- ou -S-.

**11.** Procédé selon la revendication 1, dans lequel $R^1$ est le benzothiazolyle, n est égal à 1, $R^2$ et $R^3$ sont chacun un propyle et $R^4$ est un hydrogène.

**12.** Modification du procédé revendiqué dans la revendication 1, qui est caractérisée en ce que l'on utilise un composé de la formule (I) ou un sel non toxique de celui-ci, produit par un procédé revendiqué dans la revendication 1, pour réaliser une forme pharmaceutiquement acceptable par mélange ou combinaison dudit composé avec un diluant ou vecteur pharmaceutiquement acceptable.

**Patentansprüche**

**Patentansprüche für folgende Vertragsstaaten : AT, BE, CH, DE, FR, GB, IT, LI, LU, NL, SE**

**1.** Dithioacetal-Verbindung der Formel

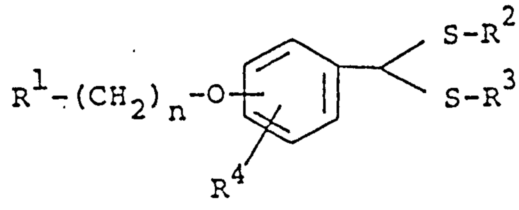

worin

$R^1$ darstellt ($C_1$-$C_6$)-Alkyl, Di-($C_1$ $C_6$)-alkylamino, Aryl ausgewählt aus der Gruppe, bestehend aus Phenyl, Tolyl, Xylyl oder Naphthyl, oder eine heterocyclische Gruppe, die ein oder mehrere Substituenten haben kann, ausgewählt unter Halogen, ($C_1$-$C_6$)-Alkyl, ($C_1$-$C_6$)-Alkoxy und Phenyl, wobei die heterocyclische Gruppe aus der Gruppe ausgewählt ist, bestehend aus einer ungesättigten 3- bis 8-gliedrigen monocyclischen heterocyclischen Gruppe, die 1 bis 4 Stickstoffatome enthält, ausgewählt aus der Gruppe, bestehend aus Pyrrolyl, Pyrrolinyl, Imidazolyl, Pyrazolyl, Pyridyl und dessen N-Oxid, Dihydropyridyl, Pyrimidyl, Pyrazinyl, Pyridazinyl, Triazolyl, einer 3- bis 8-gliedrigen monocyclischen heterocyclischen Gruppe, die wenigstens ein Schwefelatom und wenigstens ein Stickstoffatom enthält, ausgewählt aus der Gruppe, bestehend aus Thiazolyl, Isothiazolyl, Thiadiazolyl, einer polycyclischen heterocyclischen Gruppe, die wenigstens ein Stickstoffatom enthält, ausgewählt aus der Gruppe, bestehend aus Indolyl, Isoindolyl, Indolizinyl, Benzimidazolyl, Chinolyl, Isochinolyl, Chinazolinyl, Imidazo(1,2-a)pyridyl, einer polycyclischen heterocyclischen Gruppe, die wenigstens ein Schwefelatom und wenigstens ein Stickstoffatom enthält, ausgewählt aus der Gruppe, bestehend aus Benzothiazolyl, Benzothiadiazolyl, einer polycyclischen heterocyclischen Gruppe, die wenigstens ein Sauerstoffatom enthält, ausgewählt aus der Gruppe, bestehend aus Benzofuranyl, Isobenzofuranyl, einer polycyclischen heterocyclischen Gruppe, die wenigstens ein Sauerstoffatom und wenigstens ein Stickstoffatom enthält, ausgewählt aus der Gruppe, bestehend aus Benzoxazolyl, Benzoxadiazolyl, darstellt,

$R^2$ und $R^3$ sind jeweils ($C_1$-$C_6$)-Alkyl, Aryl oder Ar-($C_1$-$C_6$)-alkyl, wobei Aryl und der Arylteil ausgewählt sind aus der Gruppe, bestehend aus Phenyl, Tolyl, Xylyl oder Naphthyl, oder $R^2$ und $R^3$ bilden zusammen einen 1,3-Dithianring,

$R^4$ ist Wasserstoff oder ($C_1$-$C_6$)-Alkoxy, und

n ist 0 oder eine ganze Zahl von 1 bis 4,

sowie deren pharmazeutisch annehmbare Salze.

**2.** Verbindung nach Anspruch 1, worin
$R^1$ eine heterocyclische Gruppe ist, wie in Anspruch 1 definiert,
n ist 1,
$R^2$ und $R^3$ sind jeweils ($C_1$-$C_6$)-Alkyl, und
$R^4$ ist Wasserstoff;

**3.** Verbindung nach Anspruch 1, worin die Verbindung repräsentiert wird durch die folgende Formel

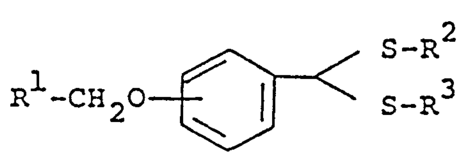

worin $R^1$ eine wie oben definierte heterocyclische Gruppe ist, die einen oder mehrere Substituenten aufweist, ausgewählt unter Halogen, ($C_1$-$C_6$)-Alkyl, ($C_1$-$C_6$)-Alkoxy und Phenyl, und $R^2$ und $R^3$ sind jeweils ($C_1$-$C_6$)-Alkyl.

**4.** Verbindung nach Anspruch 1, worin die Verbindung repräsentiert wird durch die folgende Formel

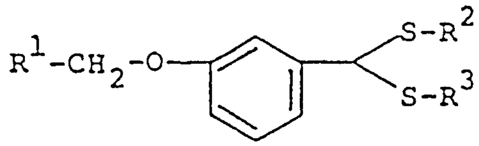

worin $R^1$ eine ungesättigte Benzen-kondensierte 5- oder 6-gliedrige heterocyclische Gruppe ist, die ein oder zwei Stickstoffatome enthält, wie in Anspruch 1 definiert, und $R^2$ und $R^3$ sind jeweils $(C_1-C_6)$-Alkyl.

**5.** Verbindung nach Anspruch 4, worin $R^1$ Chinolyl ist und $R^2$ und $R^3$ sind jeweils Propyl.

**6.** Verbindung nach Anspruch 4, worin $R^1$ eine heterocyclische Gruppe der Formel

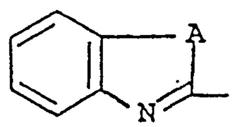

ist, worin A die Bedeutung -NH-, -O- oder -S- hat und $R^2$ und $R^3$ sind jeweils $(C_1-C_6)$-Alkyl.

**7.** Verbindung nach Anspruch 6, worin $R^1$ Benzothiazolyl ist und $R^2$ und $R^3$ sind jeweils Propyl.

**8.** Verfahren zur Herstellung einer Verbindung der folgenden Formel

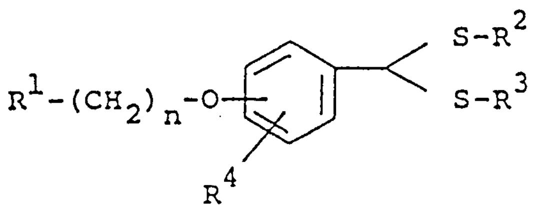

(I)

worin

$R^1$ darstellt $(C_1-C_6)$-Alkyl, Di-$(C_1$ $C_6)$-alkylamino, Aryl ausgewählt aus der Gruppe, bestehend aus Phenyl, Tolyl, Xylyl oder Naphthyl, oder eine heterocyclische Gruppe, die ein oder mehrere Substituenten haben kann, ausgewählt unter Halogen, $(C_1-C_6)$-Alkyl, $(C_1-C_6)$-Alkoxy und Phenyl, wobei die heterocyclische Gruppe aus der Gruppe ausgewählt ist, bestehend aus einer ungesättigten 3- bis 8-gliedrigen monocyclischen heterocyclischen Gruppe, die 1 bis 4 Stickstoffatome enthält, ausgewählt aus der Gruppe, bestehend aus Pyrrolyl, Pyrrolinyl, Imidazolyl, Pyrazolyl, Pyridyl und dessen N-Oxid, Dihydropyridyl, Pyrimidyl, Pyrazinyl, Pyridazinyl, Triazolyl, einer 3- bis 8-gliedrigen monocyclischen heterocyclischen Gruppe, die wenigstens ein Schwefelatom und wenigstens ein Stickstoffatom enthält, ausgewählt aus der Gruppe, bestehend aus Thiazolyl, Isothiazolyl, Thiadiazolyl, einer polycyclischen heterocyclischen Gruppe, die wenigstens ein Stickstoffatom enthält, ausgewählt aus der Gruppe, bestehend aus Indolyl, Isoindolyl, Indolizinyl, Benzimidazolyl, Chinolyl, Isochinolyl, Chinazolinyl, Imidazo(1,2-a)pyridyl, einer polycyclischen heterocyclischen Gruppe, die wenigstens ein Schwefelatom und wenigstens ein Stickstoffatom enthält, ausgewählt aus der Gruppe, bestehend aus Benzothiazolyl, Benzothiadiazolyl, einer polycyclischen heterocyclischen Gruppe, die wenigstens ein Sauerstoffatom enthält, ausgewählt aus der Gruppe, bestehend aus Benzofuranyl, Isobenzofuranyl, einer polycyclischen heterocyclischen Gruppe, die wenigstens ein Sauerstoffatom und wenigstens ein Stickstoffatom enthält, ausgewählt aus der Gruppe, bestehend aus Benzoxazolyl, Benzoxadiazolyl, darstellt,

$R^2$ und $R^3$ sind jeweils $(C_1-C_6)$-Alkyl, Aryl oder Ar-$(C_1-C_6)$-alkyl, wobei Aryl und der Arylteil ausgewählt sind aus der Gruppe, bestehend aus Phenyl, Tolyl, Xylyl oder Naphthyl, oder $R^2$ und $R^3$ bilden zusammen einen 1,3-Dithianring,

$R^4$ ist Wasserstoff oder $(C_1-C_6)$-Alkoxy, und

n ist 0 oder eine ganze Zahl von 1 bis 4, sowie deren pharmazeutisch annehmbare Salze, gekennzeichnet durch Umsetzen einer Verbindung der folgenden Formel oder deren Salz

$R^1$-$(CH_2)_n$-Y

mit einer Verbindung der folgenden Formel oder deren Salz

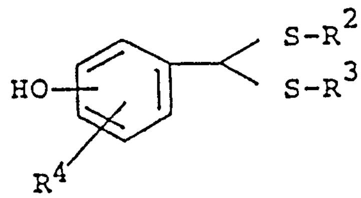

um zu einer Verbindung der folgenden Formel oder deren Salz zu gelangen

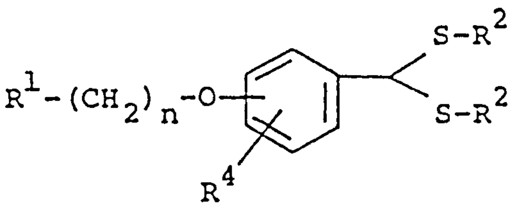

oder
Umsetzen einer Verbindung der folgenden Formel oder deren Salz

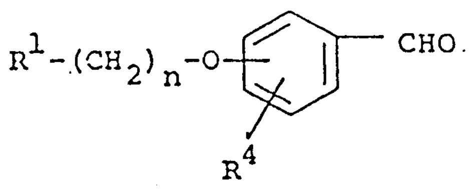

mit einer Verbindung der folgenden Formel oder deren Salz

$R^2$-SH

um zu einer Verbindung der folgenden Formel oder deren Salz zu gelangen

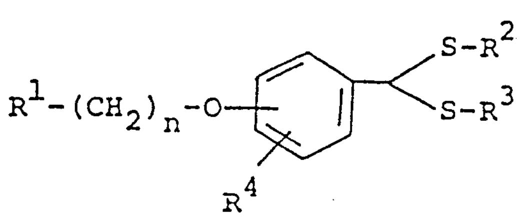

oder
Umsetzen einer Verbindung der folgenden Formel oder deren Salz

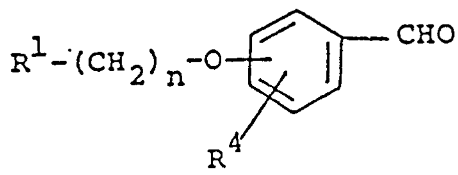

mit einer Verbindung der folgenden Formel oder deren Salz

$SH-(CH_2)_3-SH$

um zu einer Verbindung der folgenden Formel oder deren Salz zu gelangen

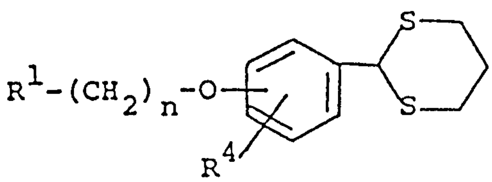

oder
Umsetzen einer Verbindung der folgenden Formel oder deren Salz

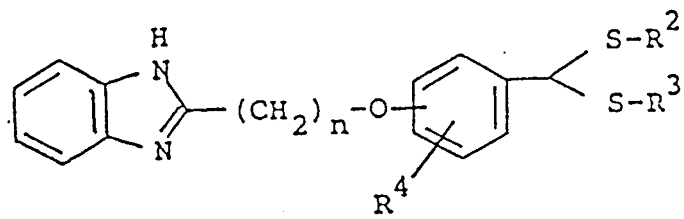

mit ($C_1$-$C_6$)-Alkyl-alkylierenden Mitteln, um zu einer Verbindung der folgenden Formel oder deren Salz zu gelangen

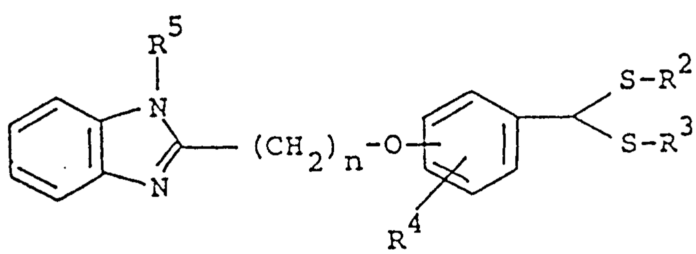

worin in den obigen Formeln $R^1$, $R^2$, $R^3$, $R^4$ und n die oben genannte Bedeutung haben, $R^5$ ist ($C_1$-$C_6$)-Alkyl und Y ist ein Säurerest, ausgewählt aus der Gruppe, bestehend aus einem anorganischen Säurerest, ausgewählt aus der Gruppe, bestehend aus Salzsäure, Bromwasserstoffsäure, Jodwasserstoffsäure, Schwefelsäure, einer organischen Säure, ausgewählt aus der Gruppe, bestehend aus einer Sulfonsäure, ausgewählt aus Methansulfonsäure, Benzensulfonsäure und Toluensulfonsäure, und Dimethylcarbaminsäure.

**9.** Verfahren nach Anspruch 8, worin $R^1$ eine heterocyclische Gruppe ist, n ist 1, $R^2$ und $R^3$ sind jeweils ($C_1$-$C_6$-Alkyl, und $R^4$ ist Wasserstoff.

10. Verfahren nach Anspruch 9, worin die heterocyclische Gruppe eine ungesättigte Benzen-kondensierte 5- oder 6-gliedrige heterocyclische Gruppe ist, enthaltend ein oder zwei Stickstoffatome, wie in Anspruch 1 definiert.

11. Verfahren nach Anspruch 8, worin $R^1$ Chinolyl ist, n ist 1, $R^2$ und $R^3$ sind jeweils Propyl, und $R^4$ ist Wasserstoff.

12. Verfahren nach Anspruch 9, worin die heterocyclische Gruppe durch die folgende Formel dargestellt wird

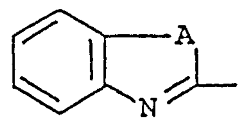

worin A die Bedeutung -NH-, -O- oder -S- hat.

13. Verfahren nach Anspruch 8, worin $R^1$ Benzothiazolyl ist, n ist 1, $R^2$ und $R^3$ sind jeweils Propyl und $R^4$ ist Wasserstoff.

14. Pharmazeutische Zusammensetzung, umfassend als einen aktiven Bestandteil eine Verbindung, wie in Anspruch 1 definiert, oder deren pharmazeutisch annehmbares Salz.

15. Antiinflammatorisches oder antiallergisches Mittel, umfassend als einen aktiven Bestandteil eine Verbindung, wie in Anspruch 1 definiert, oder deren pharmazeutisch annehmbares Salz.

16. Verbindung nach Anspruch 1 oder pharmazeutisch annehmbare Salze davon zur Verwendung als ein Medikament.

17. Verbindung nach Anspruch 1 oder pharmazeutisch annehmbare Salze davon zur Verwendung bei der Behandlung oder Verhütung entzündlicher oder allergischer Krankheiten.

18. Verwendung einer Verbindung nach Anspruch 1 oder deren pharmazeutisch annehmbaren Salzes zur Herstellung eines Medikaments zur therapeutischen Behandlung von entzündlichen oder allergischen Krankheiten.

**Patentansprüche für folgenden Vertragsstaat : ES**

1. Verfahren zur Herstellung einer Dithioacetalverbindung der Formel

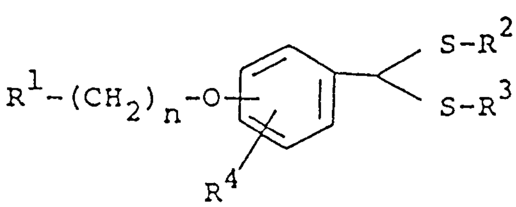

worin

$R^1$ darstellt ($C_1$-$C_6$)-Alkyl, Di-($C_1$ $C_6$)-alkylamino, Aryl ausgewählt aus der Gruppe, bestehend aus Phenyl, Tolyl, Xylyl oder Naphthyl, oder eine heterocyclische Gruppe, die ein oder mehrere Substituenten haben kann, ausgewählt unter Halogen, ($C_1$-$C_6$)-Alkyl, ($C_1$-$C_6$)-Alkoxy und Phenyl, wobei die heterocyclische Gruppe aus der Gruppe ausgewählt ist, bestehend aus einer ungesättigten 3- bis 8-gliedrigen monocyclischen heterocyclischen Gruppe, die 1 bis 4 Stickstoffatome enthält, ausgewählt aus der Gruppe, bestehend aus Pyrrolyl, Pyrrolinyl, Imidazolyl, Pyrazolyl, Pyridyl und dessen N-Oxid,

Dihydropyridyl, Pyrimidyl, Pyrazinyl, Pyridazinyl, Triazolyl, einer 3- bis 8-gliedrigen monocyclischen heterocyclischen Gruppe, die wenigstens ein Schwefelatom und wenigstens ein Stickstoffatom enthält, ausgewählt aus der Gruppe, bestehend aus Thiazolyl, Isothiazolyl, Thiadiazolyl, einer polycyclischen heterocyclischen Gruppe, die wenigstens ein Stickstoffatom enthält, ausgewählt aus der Gruppe, bestehend aus Indolyl, Isoindolyl, Indolizinyl, Benzimidazolyl, Chinolyl, Isochinolyl, Chinazolinyl, Imidazo(1,2-a)pyridyl, einer polycyclischen heterocyclischen Gruppe, die wenigstens ein Schwefelatom und wenigstens ein Stickstoffatom enthält, ausgewählt aus der Gruppe, bestehend aus Benzothiazolyl, Benzothiadiazolyl, einer polycyclischen heterocyclischen Gruppe, die wenigstens ein Sauerstoffatom enthält, ausgewählt aus der Gruppe, bestehend aus Benzofuranyl, Isobenzofuranyl, einer polycyclischen heterocyclischen Gruppe, die wenigstens ein Sauerstoffatom und wenigstens ein Stickstoffatom enthält, ausgewählt aus der Gruppe, bestehend aus Benzoxazolyl, Benzoxadiazolyl, darstellt,

$R^2$ und $R^3$ sind jeweils $(C_1$-$C_6)$-Alkyl, Aryl oder Ar-$(C_1$-$C_6)$-alkyl, wobei Aryl und der Arylteil ausgewählt sind aus der Gruppe, bestehend aus Phenyl, Tolyl, Xylyl oder Naphthyl, oder $R^2$ und $R^3$ bilden zusammen einen 1,3-Dithianring,

$R^4$ ist Wasserstoff oder $(C_1$-$C_6)$-Alkoxy, und

n ist 0 oder eine ganze Zahl von 1 bis 4,

sowie deren pharmazeutisch annehmbare Salze, gekennzeichnet durch Umsetzen einer Verbindung der folgenden Formel oder deren Salz

$R^1$-$(CH_2)_n$-Y

mit einer Verbindung der folgenden Formel oder deren Salz

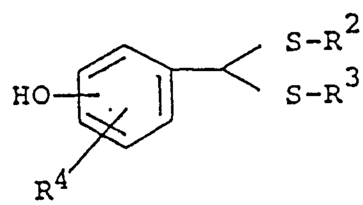

um zu einer Verbindung der folgenden Formel oder deren Salz zu gelangen

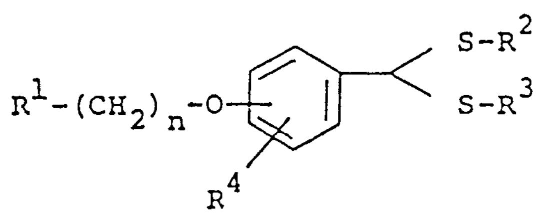

(I)

oder

Umsetzen einer Verbindung der folgenden Formel oder deren Salz

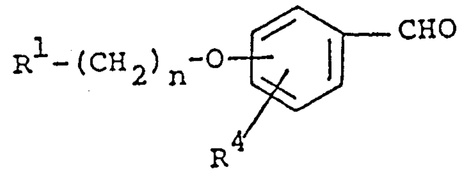

mit einer Verbindung der folgenden Formel oder deren Salz

$R^2$-SH

um zu einer Verbindung der folgenden Formel oder deren Salz zu gelangen

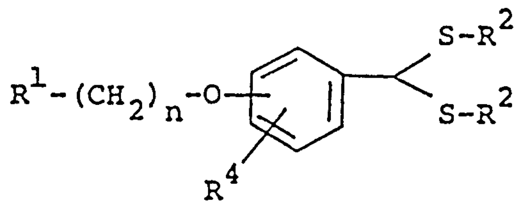

oder
Umsetzen einer Verbindung der folgenden Formel oder deren Salz

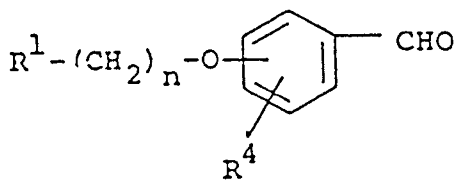

mit einer Verbindung der folgenden Formel oder deren Salz

$SH-(CH_2)_3-SH$

um zu einer Verbindung der folgenden Formel oder deren Salz zu gelangen

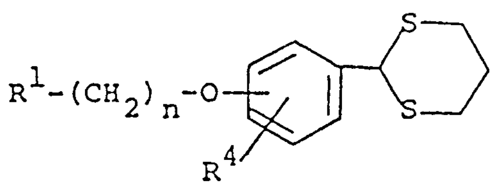

oder
Umsetzen einer Verbindung der folgenden Formel oder deren Salz

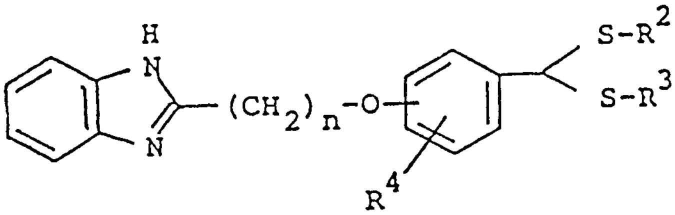

mit ($C_1$-$C_6$)-Alkyl-alkylierenden Mitteln, um zu einer Verbindung der folgenden Formel oder deren Salz zu gelangen

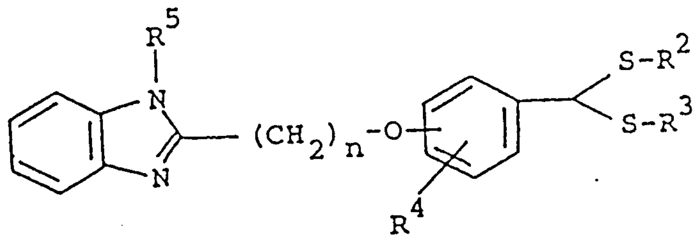

worin in den obigen Formeln $R^1$, $R^2$, $R^3$, $R^4$ und n die oben genannte Bedeutung haben, $R^5$ ist ($C_1$-$C_6$)-Alkyl und Y ist ein Säurerest, ausgewählt aus der Gruppe, bestehend aus einem anorganischen Säurerest, ausgewählt aus der Gruppe, bestehend aus Salzsäure, Bromwasserstoffsäure, Jodwasserstoffsäure, Schwefelsäure, einer organischen Säure, ausgewählt aus der Gruppe, bestehend aus einer Sulfonsäure, ausgewählt aus Methansulfonsäure, Benzensulfonsäure und Toluensulfonsäure, und Dimethylcarbaminsäure.

**2.** Verfahren nach Anspruch 1, worin $R^1$ eine wie in Anspruch 1 definierte heterocyclische Gruppe ist, n ist 1, $R^2$ und $R^3$ sind jeweils ($C_1$-$C_6$-Alkyl, und $R^4$ ist Wasserstoff.

**3.** Verfahren nach Anspruch 1, worin die Verbindung durch die folgende Formel repräsentiert wird

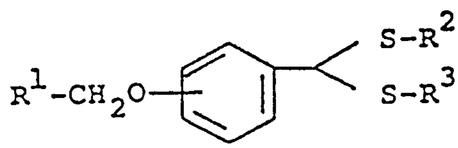

worin $R^1$ eine wie im Anspruch 1 definierte heterocyclische Gruppe ist, die einen oder mehrere Substituenten aufweist, ausgewählt unter Halogen, ($C_1$-$C_6$)-Alkyl, ($C_1$-$C_6$)-Alkoxy und Phenyl, und $R^2$ und $R^3$ sind jeweils ($C_1$-$C_6$)-Alkyl.

**4.** Verfahren nach Anspruch 1, worin die Verbindung durch die folgende Formel repräsentiert wird

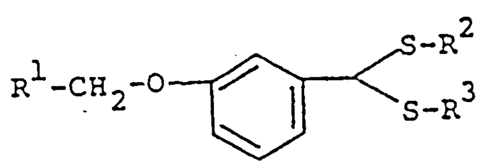

worin $R^1$ eine ungesättigte Benzen-kondensierte 5- oder 6-gliedrige heterocyclische Gruppe ist, die ein oder zwei Stickstoffatome enthält, wie in Anspruch 1 definiert, und $R^2$ und $R^3$ sind jeweils ($C_1$-$C_6$)-Alkyl.

**5.** Verfahren nach Anspruch 4, worin $R^1$ Chinolyl ist und $R^2$ und $R^3$ sind jeweils Propyl.

**6.** Verfahren nach Anspruch 4, worin $R^1$ eine heterocyclische Gruppe der Formel

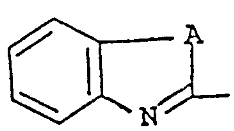

ist, worin A die Bedeutung -NH-, -O- oder -S- hat und $R^2$ und $R^3$ sind jeweils ($C_1$-$C_6$)-Alkyl.

7. Verfahren nach Anspruch 6, worin $R^1$ Benzothiazolyl ist und $R^2$ und $R^3$ sind jeweils Propyl.

8. Verfahren nach Anspruch 2, worin die heterocyclische Gruppe eine ungesättigte Benzen-kondensierte 5- oder 6-gliedrige heterocyclische Gruppe ist, enthaltend ein oder zwei Stickstoffatome, wie in Anspruch 1 definiert.

9. Verfahren nach Anspruch 1, worin $R^1$ Chinolyl ist, n ist 1, $R^2$ und $R^3$ sind jeweils Propyl, und $R^4$ ist Wasserstoff.

10. Verfahren nach Anspruch 2, worin die heterocyclische Gruppe durch die folgende Formel dargestellt wird

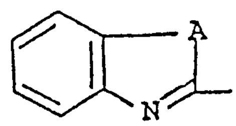

worin A die Bedeutung -NH-, -O- oder -S- hat.

11. Verfahren nach Anspruch 1, worin $R^1$ Benzothiazolyl ist, n ist 1, $R^2$ und $R^3$ sind jeweils Propyl und $R^4$ ist Wasserstoff.

**Patentansprüche für folgenden Vertragsstaat : GR**

1. Verfahren zur Herstellung einer Dithioacetalverbindung der Formel

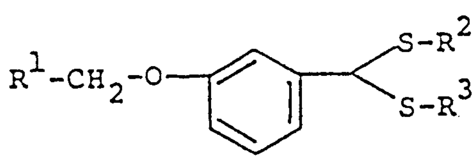

worin

$R^1$ darstellt ($C_1$-$C_6$)-Alkyl, Di-($C_1$ $C_6$)-alkylamino, Aryl ausgewählt aus der Gruppe, bestehend aus Phenyl, Tolyl, Xylyl oder Naphthyl, oder eine heterocyclische Gruppe, die ein oder mehrere Substituenten haben kann, ausgewählt unter Halogen, ($C_1$-$C_6$)-Alkyl, ($C_1$-$C_6$)-Alkoxy und Phenyl, wobei die heterocyclische Gruppe aus der Gruppe ausgewählt ist, bestehend aus einer ungesättigten 3- bis 8-gliedrigen monocyclischen heterocyclischen Gruppe, die 1 bis 4 Stickstoffatome enthält, ausgewählt aus der Gruppe, bestehend aus Pyrrolyl, Pyrrolinyl, Imidazolyl, Pyrazolyl, Pyridyl und dessen N-Oxid, Dihydropyridyl, Pyrimidyl, Pyrazinyl, Pyridazinyl, Triazolyl, einer 3- bis 8-gliedrigen monocyclischen heterocyclischen Gruppe, die wenigstens ein Schwefelatom und wenigstens ein Stickstoffatom enthält, ausgewählt aus der Gruppe, bestehend aus Thiazolyl, Isothiazolyl, Thiadiazolyl, einer polycyclischen heterocyclischen Gruppe, die wenigstens ein Stickstoffatom enthält, ausgewählt aus der Gruppe, bestehend aus Indolyl, Isoindolyl, Indolizinyl, Benzimidazolyl, Chinolyl, Isochinolyl, Chinazolinyl, Imidazo(1,2-a)pyridyl, einer polycyclischen heterocyclischen Gruppe, die wenigstens ein Schwefelatom und wenigstens ein Stickstoffatom enthält, ausgewählt aus der Gruppe, bestehend aus Benzothiazolyl, Benzothiadiazolyl, einer polycyclischen heterocyclischen Gruppe, die wenigstens ein Sauerstoffatom enthält, ausgewählt aus der Gruppe, bestehend aus Benzofuranyl, Isobenzofuranyl, einer polycyclischen heterocyclischen Gruppe, die wenigstens ein Sauerstoffatom und wenigstens ein Stickstoffatom enthält, ausgewählt aus der Gruppe, bestehend aus Benzoxazolyl, Benzoxadiazolyl, darstellt,

$R^2$ und $R^3$ sind jeweils ($C_1$-$C_6$)-Alkyl, Aryl oder Ar-($C_1$-$C_6$)-alkyl, wobei Aryl und der Arylteil ausgewählt sind aus der Gruppe, bestehend aus Phenyl, Tolyl, Xylyl oder Naphthyl, oder $R^2$ und $R^3$ bilden zusammen einen 1,3-Dithianring,

$R^4$ ist Wasserstoff oder ($C_1$-$C_6$)-Alkoxy, und

n ist 0 oder eine ganze Zahl von 1 bis 4,
sowie deren pharmazeutisch annehmbare Salze, gekennzeichnet durch Umsetzen einer Verbindung der folgenden Formel oder deren Salz

$R^1$-$(CH_2)_n$-Y

mit einer Verbindung der folgenden Formel oder deren Salz

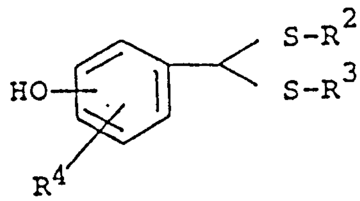

um zu einer Verbindung der folgenden Formel oder deren Salz zu gelangen

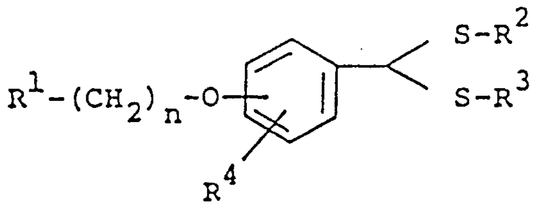

(I)

oder
Umsetzen einer Verbindung der folgenden Formel oder deren Salz

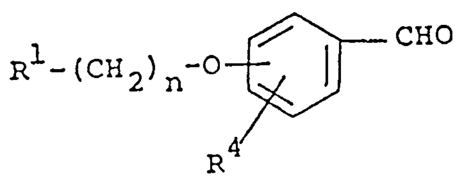

mit einer Verbindung der folgenden Formel oder deren Salz

$R^2$-SH

um zu einer Verbindung der folgenden Formel oder deren Salz zu gelangen

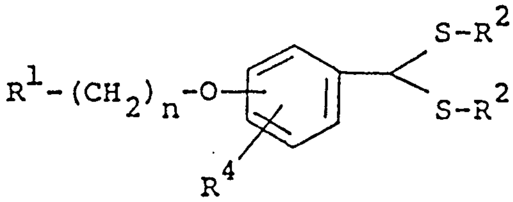

oder
Umsetzen einer Verbindung der folgenden Formel oder deren Salz

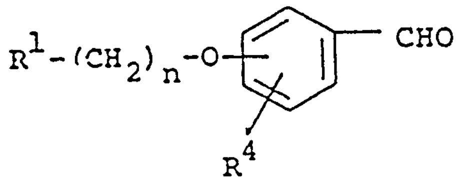

mit einer Verbindung der folgenden Formel oder deren Salz

$SH-(CH_2)_3-SH$

um zu einer Verbindung der folgenden Formel oder deren Salz zu gelangen

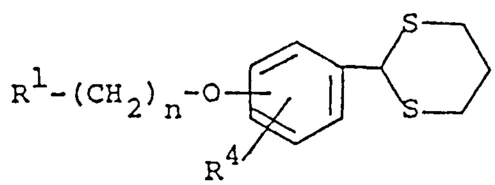

oder
Umsetzen einer Verbindung der folgenden Formel oder deren Salz

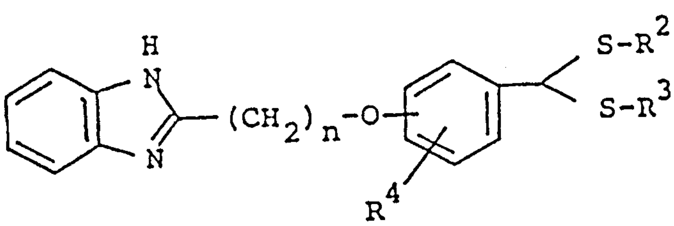

mit ($C_1$-$C_6$)-Alkyl-alkylierenden Mitteln, um zu einer Verbindung der folgenden Formel oder deren Salz zu gelangen

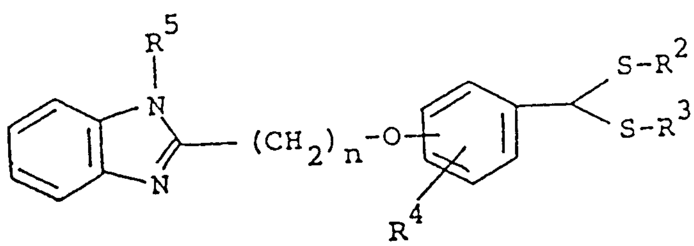

worin in den obigen Formeln $R^1$, $R^2$, $R^3$, $R^4$ und n die oben genannte Bedeutung haben, $R^5$ ist ($C_1$-$C_6$)-Alkyl und Y ist ein Säurerest, ausgewählt aus der Gruppe, bestehend aus einem anorganischen Säurerest, ausgewählt aus der Gruppe, bestehend aus Salzsäure, Bromwasserstoffsäure, Jodwasserstoffsäure, Schwefelsäure, einer organischen Säure, ausgewählt aus der Gruppe, bestehend aus einer Sulfonsäure, ausgewählt aus Methansulfonsäure, Benzensulfonsäure und Toluensulfonsäure, und Dimethylcarbaminsäure.

**2.** Verfahren nach Anspruch 1, worin $R^1$ eine wie in Anspruch 1 definierte heterocyclische Gruppe ist, n ist 1, $R^2$ und $R^3$ sind jeweils ($C_1$-$C_6$-Alkyl, und $R^4$ ist Wasserstoff.

**3.** Verfahren nach Anspruch 1, worin die Verbindung durch die folgende Formel repräsentiert wird

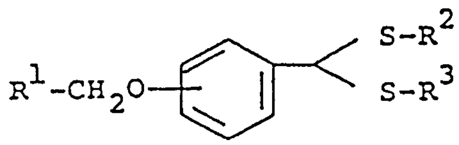

worin $R^1$ eine wie im Anspruch 1 definierte heterocyclische Gruppe ist, die einen oder mehrere Substituenten aufweist, ausgewählt unter Halogen, $(C_1$-$C_6)$-Alkyl, $(C_1$-$C_6)$-Alkoxy und Phenyl, und $R^2$ und $R^3$ sind jeweils $(C_1$-$C_6)$-Alkyl.

4. Verfahren nach Anspruch 1, worin die Verbindung durch die folgende Formel repräsentiert wird

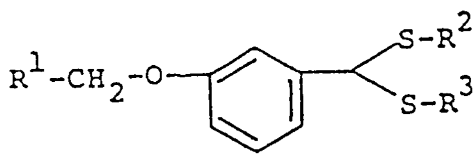

worin $R^1$ eine ungesättigte Benzen-kondensierte 5- oder 6-gliedrige heterocyclische Gruppe ist, die ein oder zwei Stickstoffatome enthält, wie in Anspruch 1 definiert, und $R^2$ und $R^3$ sind jeweils $(C_1$-$C_6)$-Alkyl.

5. Verfahren nach Anspruch 4, worin $R^1$ Chinolyl ist und $R^2$ und $R^3$ sind jeweils Propyl.

6. Verfahren nach Anspruch 4, worin $R^1$ eine heterocyclische Gruppe der Formel

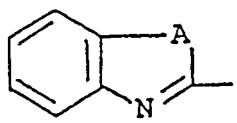

ist, worin A die Bedeutung -NH-, -O- oder -S- hat und $R^2$ und $R^3$ sind jeweils $(C_1$-$C_6)$-Alkyl.

7. Verfahren nach Anspruch 6, worin $R^1$ Benzothiazolyl ist und $R^2$ und $R^3$ sind jeweils Propyl.

8. Verfahren nach Anspruch 2, worin die heterocyclische Gruppe eine ungesättigte Benzen-kondensierte 5- oder 6-gliedrige heterocyclische Gruppe ist, enthaltend ein oder zwei Stickstoffatome, wie in Anspruch 1 definiert.

9. Verfahren nach Anspruch 1, worin $R^1$ Chinolyl ist, n ist 1, $R^2$ und $R^3$ sind jeweils Propyl, und $R^4$ ist Wasserstoff.

10. Verfahren nach Anspruch 2, worin die heterocyclische Gruppe durch die folgende Formel dargestellt wird

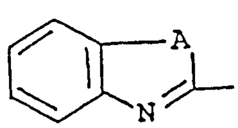

worin A die Bedeutung -NH-, -O- oder -S- hat.

11. Verfahren nach Anspruch 1, worin $R^1$ Benzothiazolyl ist, n ist 1, $R^2$ und $R^3$ sind jeweils Propyl und $R^4$

ist Wasserstoff.

**12.** Modifikation des Verfahrens nach Anspruch 1, gekennzeichnet durch Bringen einer Verbindung der Formel (I) oder eines nicht-toxischen Salzes davon, hergestellt durch ein Verfahren nach Anspruch 1, in eine pharmazeutisch annehmbare Form mittels Beimischen oder Bereitstellung der Verbindung mit einem pharmazeutisch annehmbaren Verdünnungsmittel oder Träger.